# EUROPEAN PATENT APPLICATION

(11) **EP 2 727 577 A1**
(43) Date of publication of application: **07.05.2014**
(21) Application number: 13191438.4
(22) Date of filing: 06.05.2008
(51) Int. Cl.: A61K 8/19, A61K 8/25, A61K 8/26, A61K 8/27, A61K 8/29, A61Q 1/06, A61Q 1/10, A61Q 1/12, C09C 3/08, B82Y 30/00, C09C 3/00, A61K 8/28

(54) **Colour pigment powder, pigment dispersion and method of manufacture**

(30) Priority: 07.05.2007 US 928146 P
(62) Divisional of application: 08767639.1
(71) Applicant: U.S. Cosmetics Corporation, Dayville CT 06241-0859 (US)
(72) Inventor: Kishida Shigeru, Storrs, CT 06268 (US); Kawasaki, Yoshiaki, Woodstock, CT 06281 (US); Lepage, Mark, George, Dudley, MA 01571 (US); Weaver, Lafrancia, Shree, Dayville, CT 06241 (US)
(74) Representative: Campbell, Patrick John Henry

(57) **Abstract**

The invention provides, among other things, fully and partially extended color bulk powders and partially and fully extended color bulk dispersions. Invention fully and partially extended color powders and fully and partially extended color dispersions can be used in cosmetic and makeup products, personal care products, and pharmaceutical products.

## Description

### RELATED APPLICATIONS

This application claims priority to U.S. provisional application Serial No. 60/928,146, filed May 7, 2007, which is herein incorporated by reference in its entirety for all purposes.

### FIELD OF THE INVENTION

This invention relates to fully extended color powder materials and dispersions, and methods of making fully extended color powder materials and dispersions useful for cosmetic products such as foundations, eye shadows, lip colors, mascaras, lotions, and creams.

### INTRODUCTION

Current cosmetics and make-ups on market employ hydrophobically modified pigments and substrates as components for establishing excellent resistance against sebum and sweat. The conventional manufacturing process for preparing cosmetic and make-up products includes mixing pigments, extenders and/or other substrates and then atomizing them until the colors are well developed. Oily components and auxiliary materials are added to the mixed powder, and then it is atomized to spread the oily components evenly onto the powder for making powder products. For liquid foundations, the mixed powder is dispersed into oily components by a homogenizer. This process requires many hours to pass the powder components through the atomizer several times to deagglomerate pigments and extend color. If color adjustment is needed for the product, the same procedure has to be done for adding small amounts of color pigments to the formula, which means more processing time is required.

Existing cosmetic and make-up products exhibit, for example, uneven color; color change on the skin after the application (the shade on the skin appears different from the shade of the cosmetic product prior to application); color streaks; low skin adhesion; shade changes on the skin over time (not long lasting); uneven wear or irregular spreadability of the cosmetic or makeup; and easy breakage of the pressed cakes (not strong enough).

### SUMMARY

This invention relates to fully and partially extended color bulk powders and bulk dispersions. Fully and partially extended color bulk powders include at least one pigment and at least one substrate, wherein the pigment or substrate has a surface that has been chemically immobilized with at least one surface-treatment agent; and wherein the pigment adheres to the substrate. Fully and partially extended color bulk dispersions include at least one pigment and at least one substrate, wherein the pigment or substrate has a surface that has been chemically immobilized with at least one surface-treatment agent; wherein the pigment adheres to the substrate, and wherein the material is dispersed in a liquid medium.

This invention also relates to methods for preparing fully and partially extended color bulk powders and fully and partially extended color bulk dispersions. A method for preparing a fully or partially extended color bulk powder includes providing at least one pigment and a substrate; contacting the substrate or pigment with a surface-treatment agent to produce a surface-modified substrate or pigment material, thereby producing a substrate having adhered thereto the pigment; and blending the material until it is fully or partially extended. A method for preparing a fully or partially extended color bulk dispersion includes providing at least one pigment and a substrate; contacting the substrate or pigment with a surface-treatment agent to produce a surface-modified substrate or pigment material, thereby producing a substrate having adhered thereto the pigment; blending the material until it is fully or partially extended, and dispersing the blended material in a liquid medium.

The extent to which color bulk powders and dispersions are extended can be ascertained, for example, by determining a change or shift in color, shade, hue, chroma (saturation) or lightness upon mixing or blending. In a particular non-limiting example, a change in color is determined by the formula [(Lₙ₊₁-Lₙ)² + (aₙ₊₁-aₙ)² +(bₙ₊₁-bₙ)²]^{1/2}, which provides a ΔE value. A fully extended color bulk powder has ΔE value of less than about one, as determined by the formula [(Lₙ₊₁-Lₙ)² + (aₙ₊₁-aₙ)² + (bₙ₊₁-bₙ)²]^{1/2}, after the powder is blended. A partially extended color bulk powder has a ΔE value of less than about 2.0, as determined by the formula [(Lₙ₊₁-Lₙ)² + (aₙ₊₁-aₙ)² + (bₙ₊₁-bₙ)²]^{1/2}, after the powder is blended.

### BRIEF DESCRIPTION OF THE DRAWINGS

**Figure 1** is a schematic diagram of a substrate with different colored pigments randomly distributed on the substrate surface.

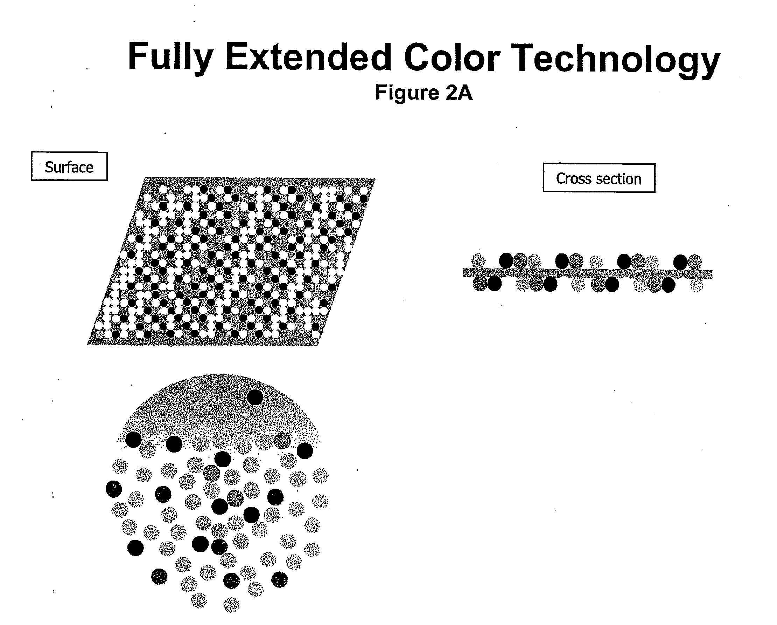

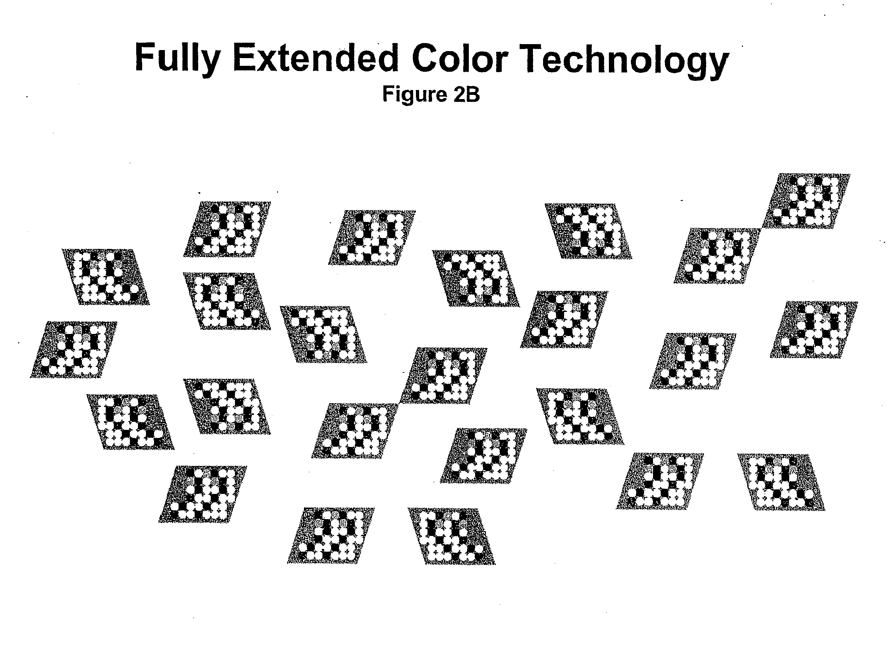

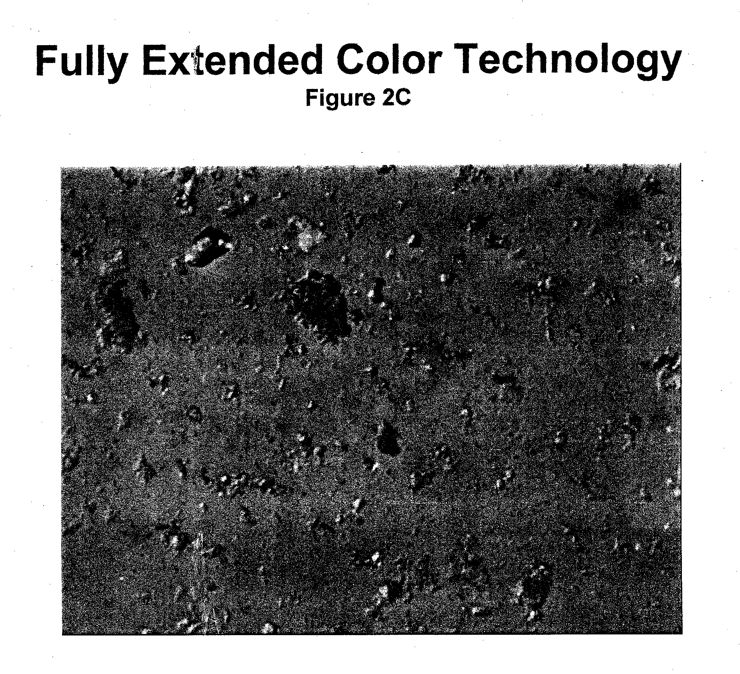

**Figures 2A-2C** are schematic diagrams of **A)** a single substrate of exemplary invention fully extended color bulk powder; **B)** a plurality of substrates of exemplary invention fully extended color bulk powder with different colored pigments randomly distributed on the substrate surface; and **C)** a photomicrograph of substrates of exemplary invention fully extended color bulk powder with different colored pigments distributed on substrate surface (600x magnification).

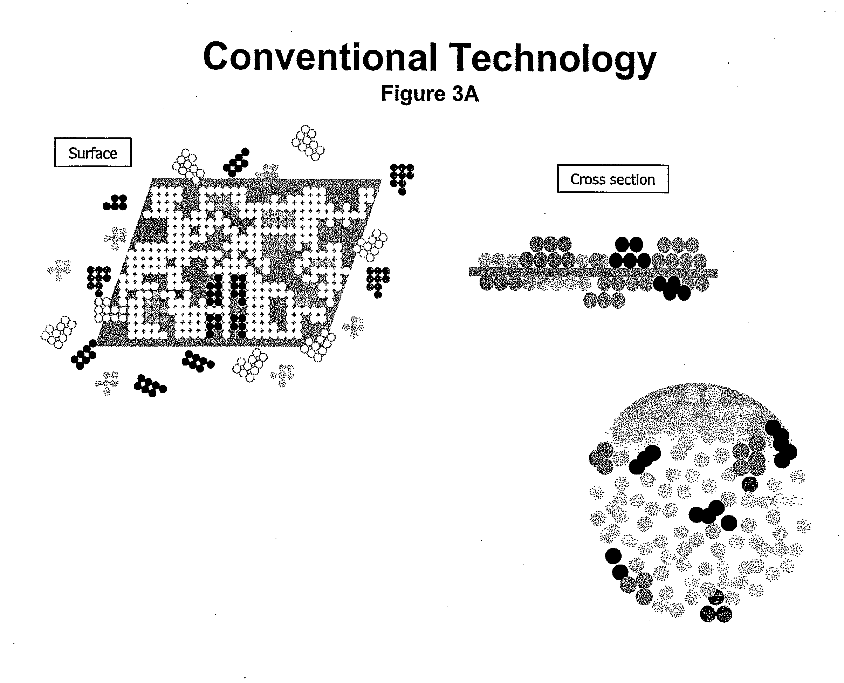

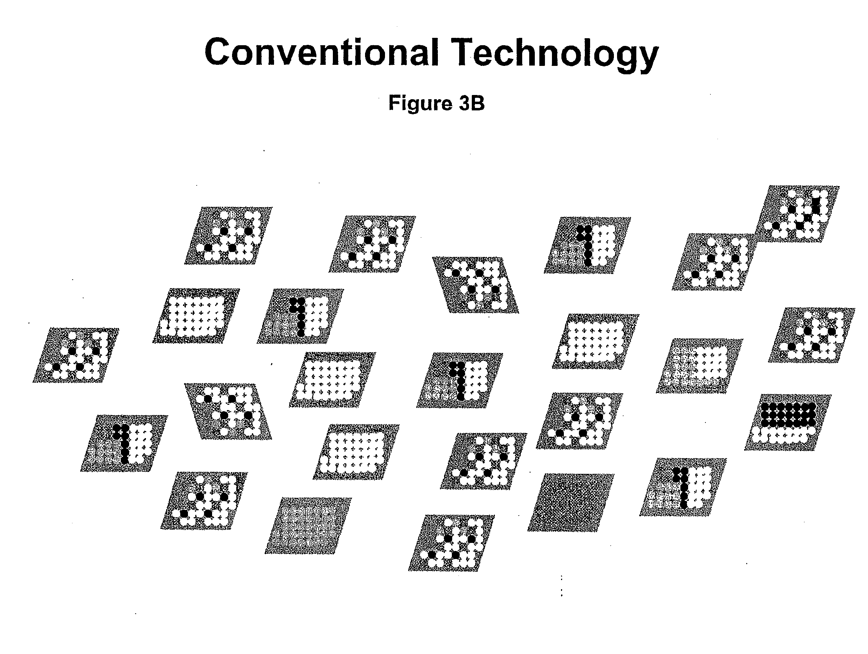

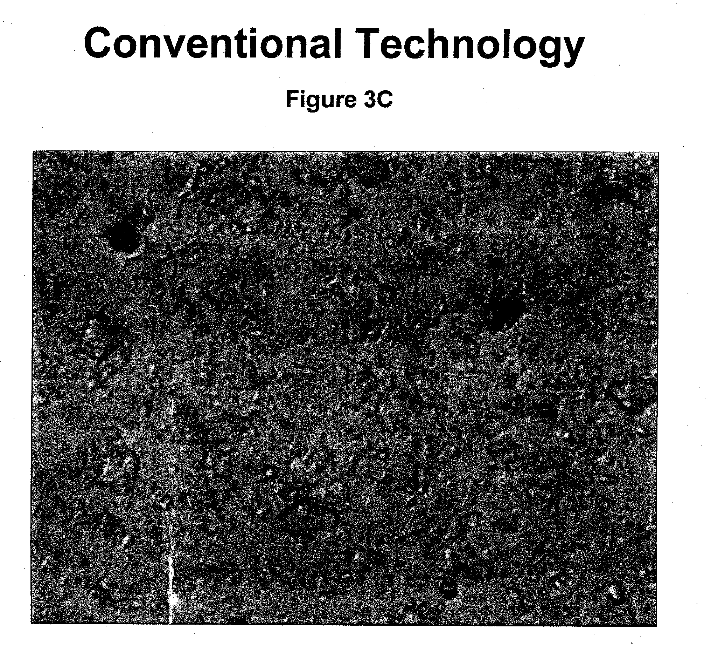

**Figures 3A-3C** are schematic diagrams of **A)** a single substrate of conventional color technology; **B)** a plurality of substrates of conventional color technology with colored pigments, including aggregates or agglomerates, that surround or are distributed unevenly, irregularly and/or non-uniformly on substrate; and **C)** a photomicrograph of conventional color technology substrates (480x magnification).

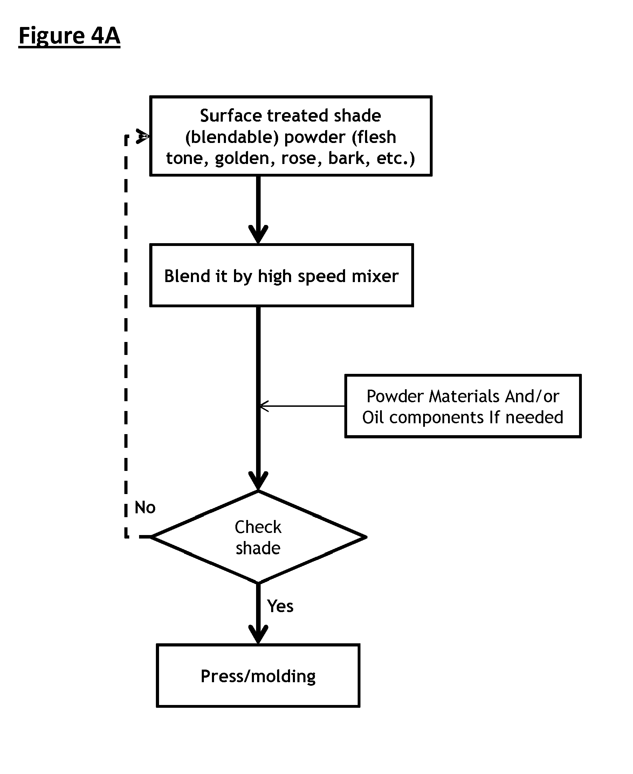

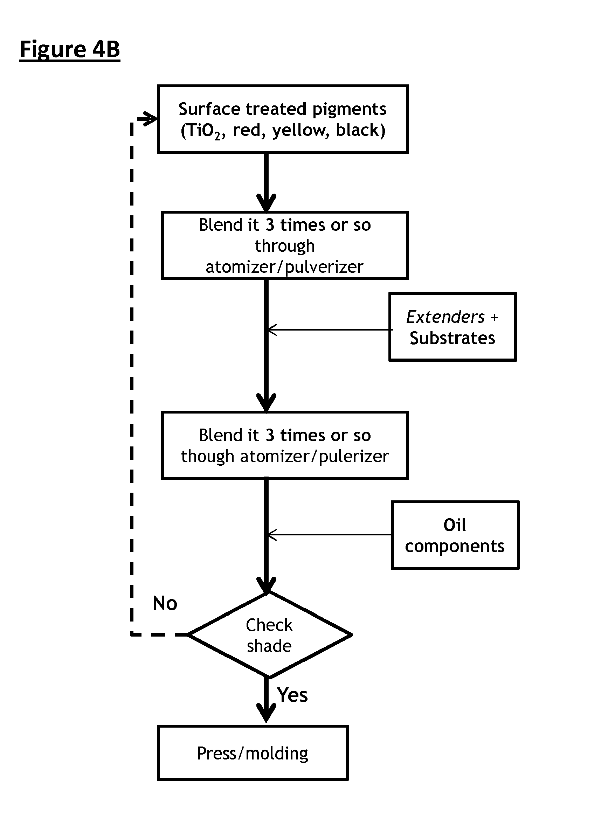

**Figures 4A** **and** **4B** show comparison flowcharts of **A)** producing fully extended color bulk powders and dispersions; and **B)** conventional manufacturing process.

### DETAILED DESCRIPTION

The invention provides, among other things, fully and partially extended color bulk powders and partially and fully extended color bulk dispersions. Invention fully and partially extended color powders and fully and partially extended color dispersions can be used in cosmetic and makeup products, personal care products, and pharmaceutical products. Fully and partially extended color bulk powders and fully and partially extended color bulk dispersion can therefore be referred to as a cosmetic, makeup, personal care or pharmaceutical precursor, since they are typically included in, further modified or materials added to produce a finished cosmetic, makeup, personal care or pharmaceutical product. For example, a fully and partially extended color powder of a single shade (e.g., fair shade) can be included in a cosmetic or makeup, personal care, or pharmaceutical product. Fully and partially extended color powders of different shades (e.g., light pink, dark yellow, yellowish red, dark brown, etc., shades) can be blended and then subsequently included in a cosmetic or makeup, personal care, or pharmaceutical product.

The term "fully extended," when used in reference to a color bulk powder or color bulk dispersion means that little or no change or shift in color, shade, hue, chroma (saturation) or lightness occurs when blending the powder or dispersion, for example, through an atomizer or pulverizer. Thus, because the color bulk powder or color bulk dispersion exhibits little or no change or shift in color, shade, hue, chroma (saturation) or lightness when blending, no additional blending or adjustment of color, shade, hue, chroma (saturation) or lightness in a fully extended color bulk powder or color bulk dispersion is required before formulation or inclusion in a cosmetic, makeup, personal care or pharmaceutical product because color, shade, hue, chroma (saturation) or lightness of the bulk powder or bulk dispersion is fully extended.

Fully extended color bulk powders and fully extended color bulk dispersion provide various superior properties over existing cosmetic, makeup, and personal care products. For example, fully extended color bulk powders and fully extended color bulk dispersions have a relatively stable color, shade, hue, chroma (saturation) or lightness, and can resist a change or shift in color, shade, hue, chroma (saturation) or lightness before and after applying to skin; streaking; shade change on skin over time; uneven or irregular spreadability that can lead to uneven wear of the cosmetic or makeup; and easy breakage of pressed cakes. Thus, fully extended color bulk powders and fully extended color bulk dispersions can maintain color, shade, hue, chroma (saturation) or lightness consistency when applied to the skin- the appearance of color, shade, hue, chroma (saturation) or lightness prior to applying to skin typically does not substantially change after initially applying to the skin, or after a period of time after applying to the skin (e.g., 1, 2, 3, 4-6, 6-12, or more hours). Additional non-limiting examples of such properties include: a more even distribution when applied to the skin and a more stable color, shade, hue, chroma (saturation) or lightness that is less susceptible to loss or streaking. Further non-limiting examples of such properties include: improved spreadability, which leads to less creasing after applying to skin.

Fully extended color bulk powders and fully extended color bulk dispersions are characterized by little if any change in one or more of color, shade, hue, chroma (saturation) or lightness upon blending, mixing, pulverizing or atomizing the powder or dispersion. In contrast, a color bulk powder or a color bulk dispersion that is not fully extended can be characterized as undergoing a detectable change in one or more of color, shade, hue, chroma (saturation) or lightness upon blending, mixing, pulverizing or atomizing the color bulk powder or dispersion. A color bulk powder or a color bulk dispersion that is partially extended is also characterized as exhibiting a detectable change or shift in one or more of color, shade, hue, chroma (saturation) or lightness upon blending, mixing, pulverizing or atomizing the color bulk powder or dispersion, but the change or shift that occurs in one or more of these parameters is less than that of a not fully extended color bulk powder or color bulk dispersion. For example, as discussed herein, the degree of color bulk powder or color bulk dispersion extension can be evaluated by measuring a change in color, shade, hue, chroma (saturation) or lightness after blending, mixing, pulverizing or atomizing-a color bulk powder or a color bulk dispersion. A partially extended color bulk powder or color bulk dispersion will undergo less of a change in color, shade, hue, chroma (saturation) or lightness after blending, mixing, pulverizing or atomizing than a not fully extended color bulk powder or color bulk dispersion.

Various methods can be used to determine whether a color bulk powder or a color bulk dispersion is fully extended, partially extended or not extended. For example, the degree of extension of a color bulk powder or a color bulk dispersion can be evaluated by measuring color, shade, hue, tint, saturation or luminance before and after blending, mixing, pulverizing or atomizing the powder or dispersion. As a non-limiting example, color of a powder can be initially measured and values assigned to L*, a* and b*, in L-a-b color coordinate system, denoted L₀*, a₀* and b₀*. L* represents lightness, with a range of 0-100; a* represents the green to red spectrum, wherein -a* is green and +a* is red; and b* represents the yellow to blue spectrum, wherein -b* is blue and +b* is yellow (see, Example 2). The powder is then blended as described and color measured again to obtain L*, a* and b* values, denoted L₁*, a₁* and b₁*. Repeating the process of blending and measuring color, to obtain L*, a* and b*values, which are assigned and the minimum number of passes (blending steps) required to obtain a color powder that exhibits little or no color change is calculated as ΔEn, which is [(Lₙ₊₁-Lₙ)² + (aₙ₊₁-aₙ)² + (bₙ₊₁-bₙ)²]^{1/2}. A ΔE value of less than about one (ΔE<1.0) means that the color is fully extended, or that little or no difference in color before and after the powder is blended. A ΔE value between 1.0 and 2.0 (ΔE=1.0-2.0) means that the color is partially extended. Typically, a partially extended color bulk powder or dispersion will have a dE value of less than about one and one-half (ΔE<1.5). A ΔE value greater than 2.0 (ΔE>2.0) means that the color is not fully extended.

Typically, a color bulk powder or dispersion that is not fully extended lacks development of one or more of color, shade, hue, chroma (saturation) or lightness as compared to a fully extended color powder or dispersion. A not fully extended color powder or dispersion therefore requires additional components (e.g., pigments, substrates, oils, etc.) or processing steps (e.g., dispersing pigments or substrates by an atomizer, pulverizer, or three-roll-mill) in order to provide a stable color, shade, hue, chroma (saturation) or lightness that is not typically required or is optional for a fully extended color bulk powder or fully extended color bulk dispersion.

Extended color bulk powder or extended color bulk dispersion can be blended together in order to produce a different color, shade, hue, chroma (saturation) or lightness. For example, to adjust or change the color, shade, hue, chroma (saturation) or lightness of a fully or partially extended color powder or dispersion, a particular color, shade, hue, chroma (saturation) or lightness can be mixed with one or more other powder(s) having a different color, shade, hue, chroma (saturation) or lightness by blending together in a mixer. If two or more fully extended color bulk powders or fully extended color dispersions are to be mixed with each other, they may be blended in a mixer to produce a desired color, shade, hue, chroma (saturation) or lightness- there is no need to blend the mixture through an atomizer, pulverizer or three roll mill. Thus, fully extended color bulk powders and color bulk dispersions of the invention can be adjusted for color, shade, hue, chroma (saturation) or lightness by simple blending without requiring multistep processing (e.g., atomizing, pulverizing), and are therefore more readily formulated or included in final cosmetic, makeup, personal care and pharmaceutical products.

Fully and partially extended color bulk powders and color bulk dispersions comprise powder materials, which include substrates and pigments acceptable for formulation or inclusion in a final cosmetic, makeup, personal care or pharmaceutical product. Thus, a fully or partially extended color bulk powder or fully or partially extended color bulk dispersion can have one or more, or all substrates or pigment or other cosmetically acceptable components suitable for formulation or inclusion in a final cosmetic, makeup, personal care or pharmaceutical product.

Substrates and pigments typically comprise or consist of a material compatible or acceptable for cosmetic and makeup products, personal care products and pharmaceutical products. Substrates and pigments are typically in the form of a powder, which is a solid, dry material consisting of extremely small, flowable particles. Particular classes of powder materials are inorganic and organic particles, beads, crystals, clays, metals, metal oxide powders, plastics and fillers for plastic suitable for cosmetic use.

A dispersion is a mixture in which a material is distributed or diffused in a medium, such as a liquid, solid or gas. A liquid is a smooth, amorphous substance in the fluid state of matter having no particular fixed shape (free flowing) and relatively invariable volume. Dispersions include an oil liquid, oil in water phase (o/w) and water in oil phase (w/o). Oil in water phase (o/w), water in oil phase (w/o) and water in silicone dispersions are typically referred to as emulsions.

Fully and partially extended color bulk powders and dispersions include at least one substrate and one pigment. Fully and partially extended color bulk powders and dispersions also include a plurality or mixture of different substrates, a plurality or mixture of different pigments, or a plurality or mixture of substrates and pigments.

Typical substrate sizes are about 1-30 microns in diameter, usually not less than 1 micron, for example, have a primary size of about 1-3 microns. Substrate particles are typically larger than pigment particles and have various shapes, for example, spherical, elliptical or "platy." Substrates provide desirable texture and other characteristics such as smoothness, silkiness, round feel, moisture feel, optical benefits (soft focusing, hiding or concealing wrinkles or blemishes), etc.

Specific non-limiting examples of substrates include clay, mica (e.g., pearl colored mica, such as Timron Super Silver^{™}, a mica coated with titanium dioxide produced by Rona/EMD Industries), talc, kaolin, sericite, silica (e.g., silica beads such as aluminum silicate, magnesium silicate and calcium sodium silicate, beadyl beads^{™}, fumed silica), alumino-silicate minerals (zeolites), nylon (e.g., nylon beads or nylon powder), acrylates such as polymethyl methacrylate (PMMA or powder), metal powders (such as aluminum), ceramic powders (such as silicon nitride or boron nitride), cotton powder, wool powder, silk powder, cellulose and cellulose powder, urethane, polystyrene and polystyrene powder, polyolefin, polyethylene and polyethylene powder, polyamide, zirconium, aluminum oxide, zirconium oxide, starch, starch powder and starch derivatives such as aluminum starch octenylsuccinate, and calcium carbonate (chalk).

Substrates include "extenders." An extender can function as a filler or bulking agent for powders and dispersions as set forth herein or known to the skilled artisan (e.g., pressed foundation, loose powder, blush, consealer, etc.). Extenders as a class typically have a size, shape or structure that is similar or identical to substrates as disclosed herein and understood by the skilled artisan. The term extender is typically used to refer to a substrate material that is added to a color bulk powder or color bulk dispersion after surface treatment or modification of pigment or substrate.

Extenders include natural and synthetic substrates that may or may not have a color, shade, hue, chroma (saturation) or lightness that may vary in saturation and luminance. As with a substrate, an extender has a size typically greater than 1 micron (1 µm), for example about 1-30 microns, and can have various shapes, for example, spherical, elliptical or "platy."

Non-limiting examples of extenders include talc, kaolin (clay), natural and synthetic micas including muscovite mica and sericite, titanated mica, cotton powder, starch, magnesium carbonate, calcium carbonate, aluminum silicate, magnesium silicate, calcium silicate, synthetic silicates, clay, bentonite, montmorillionite, calcite, chalk, bismuth oxychloride, boron nitride, fumed silica, silica beads, plastic beads such as acrylics, nylons such as Nylon 12, nylon beads, aluminum, calcium, or sodium silicate, and barium sulfate.

Amounts of substrate in fully and partially extended color bulk powders and color bulk dispersions, intermediates thereof and preparation methods of the invention will vary depending upon the precursor or intermediate product to be produced, the ultimate or final cosmetic, makeup, personal care or other product to be produced, or method of manufacture. In a fully or partially extended color bulk powder, weight percent of a substrate is typically about 0 to 95%. In a fully or partially extended color bulk dispersion, weight percent of a substrate is typically about 0 to 95%.

Although not wishing to be bound by theory, many pigment particles typically adhere to a substrate particle when pigment size is smaller than substrate size. In situations where pigment size is larger than substrate size, for example pearl pigments can be from about 50-100 microns in size with a platy structure, many substrate particles can adhere to a pigment particle. The term "adhere" used herein refers to either situation. Thus, the terminology "pigment adheres to the substrate" also includes "substrate adheres to the pigment."

Pigment adhered to substrate and substrate adhered to pigment show skin shades. Pigments adhered onto substrates or substrates adhered onto pigments may be uniformly (evenly) or non-uniformly (unevenly) distributed.

As used herein, the term "pigment," which includes "dyes" is natural or synthetic material that has a certain color, shade, hue, chroma (saturation) or lightness. Pigments may be organic or inorganic in chemical nature. Pigments typically have a primary particle diameter not greater than about 3 microns. Pigments more typically are about one order of magnitude smaller in size than substrates, for example, about 0.1-1.0 microns in diameter. Other pigments, such as pearl pigments typically have a larger size, for example 10, 20, 30, 40, or 50-100 microns (µm).

Non-limiting examples of inorganic pigments include white titanium dioxide pigments (e.g., rutile, anatase, and ultrafine TiO₂), zinc oxides (e.g., ultrafine ZnO), which can be of pigment grade and have a primary size of about 0.3 µm, or ultrafine grade, and have a primary size of less than about 0.1 µm Other inorganic pigments include zirconium oxide, zirconium dioxides, iron oxides (including yellow, red, brown, green and black iron oxides), ultramarines (such as ultramarine blue, ultramarine violet, ultramarine pink, etc.), pearl pigments (e.g., mica, titanated mica, bismuth oxychloride, etc.), manganese violet, Prussian blue, chromium oxides, chromium hydroxides, and carbon black. Non-limiting examples of organic pigments include "lake" dyes, β-carotene, carmine, chlorophyll and the like.

Fully and partially extended color bulk powders and color bulk dispersions typically include one or more different pigments. A plurality of different pigments (e.g., 2, 3, 4, 5, 6, 7, 8, 9, 10 or more pigments that optionally have a different color, shade, hue, chroma (saturation) or lightness) can be included to produce a "composite" of pigments. A plurality of different pigments, optionally having a different color, shade, hue, chroma (saturation) or lightness, can therefore be included in fully and partially extended color bulk powders and color bulk dispersions. Such extended color bulk powders and extended color bulk dispersions can be conveniently referred to as "composite" extended color bulk powders and extended color bulk dispersions.

Amounts of pigments and dyes to employ in fully and partially extended color bulk powders and color bulk dispersions, intermediates thereof and production methods of the invention will vary depending upon the desired color, shade, hue, chroma (saturation) or lightness. As set forth in Example 2, pigment types, absolute amounts and relative ratios span a broad range and can be selected based upon a desired color, shade, hue, chroma (saturation) or lightness, physical (e.g., size, shape), functional or chemical characteristic or property. In a fully or partially extended color bulk powder, total weight percent of pigments is typically about 3 to 20%, 5 to 20%, 5 to 15%, 5 to 18% or 8 to 12%. In a fully or partially extended color bulk dispersion, total weight percent of a substrate is typically about 3 to 20%, 5 to 20%, 5 to 15%, 5 to 18% or 8 to 12%.

Ratios of pigments and substrates can also vary depending upon the precursor product to be produced, the ultimate cosmetic, makeup, personal care or other product to be produced, or method of manufacture. Exemplary pigment to substrate ratio in a cosmetic or makeup product is from about 5:95 to 95:5. In personal care and other products, the ratio is typically not limited.

Substrates and pigments can be deflocculated or deagglomerated. The invention therefore provides fully and partially extended color bulk powders and color bulk dispersions, intermediates thereof and production methods that include or employ deflocculated and/or deagglomerated substrates, pigments and other cosmetically suitable materials. Substrates, and pigments can be deflocculated or deagglomerated by any physical or chemical means which achieves at least some degree of dispersal of aggregates or agglomerates. Non-limiting examples of physical deagglomerating include shearing and grinding.

As used herein, the term "surface-treatment agent" refers to chemical agents that have the ability to modify, alter or react with the surface of a powder material by forming chemical bonds on the surface of the powder. Specific non-limiting classes of surface treatment agents include surface active agents, which include surfactants, detergents, wetting agents and emulsifiers. Surface-active agents may be nonionic, anionic, cationic, amphoterics, hydrophobic or hydrophilic.

Surface-treatment agents typically have one or more reactive groups, such as a hydrophilic moiety (e.g., a carboxyl group, a phosphorous group, a sulfur group, a silanol group or a silane group) or hydrophobic moiety (e.g., a hydrocarbon, a dialkyl(CH₃-, C₂H₅-) polysiloxane, perfluoroalkyl, etc.) in their structure. Surface-treatment agents may or may not contain one or more hydroxyl groups or alkylene oxide moieties, such as ethylene oxide or propylene oxide. Those having hydroxy groups in their structure and hydrophilic characteristics can be delivered after completing the reaction onto the surface.

Non-limiting examples of surface treatment agents include acyl collagens, ether carboxylic acids, lactic acid, gluconic acid, galacturonic acid, glucarolactone, gallic acid, glucoheptanoic acid, amino acids (such as thereonine and serine) and their salts, acyl amino acids (such as acylglutamates, acylsarcosinates, acylglycinates, and acylalaninates), fatty acids and their salts, and glycerol phosphate esters (such as lecithin). Additional non-limiting examples of surface-treatment agents include methicone, dimethicone and polyethylenes with free carboxylic acids.

Examples of anionic surface active agents (surfactants) include soaps (fatty acids/ alkyl carboxylic acids salt), hydroxy fatty acids, alkyl sulfate, alkyl ether phosphate, polyoxyalkylene alkyl ether sulfate, polyoxyalkylene alkyl ether carboxylate, alkylether phosphate, acyl N-methyl taurate, N-acylamino acid salts (glutamate, sarcosinate, lalaninate, glycinate, B-alaninate), acyl peptides (acyl collagen, acyl silk protein), sodium cocoate, stearic acid, iso-stearic acid, potassium palmitate, sodium laurate, 12-hydroxystearic acid, sodium lauryl sulfate, sodium myristyl phosphate, sodium myristoyl sarcosinate, sodium polyoxyethylene lauryl sulfate, polyoxyethylene myristyl carboxylate, potassium myristate, zinc gluconate, isostearyl sebacic acid, sodium myristoyl taurate, disodium stearoyl glutamate, disodium cocoyl glutamate, arginine lauryl glycinate, sodium dilauramidoglutamide lysine.

Exemplary fatty acid surface treatment agents include structures and salts of [Formula I]: 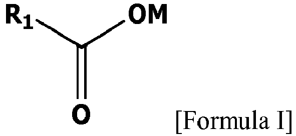
wherein R₁ is selected from the group consisting of alkyl, alkylamide, alkenyl, alkynyl, alkoxy, aryl, cycloalkyl, and arylalkyl group, all of which may be substituted by one or more hydroxy group, and may further be substituted by one or more alkoxyl, carboxyl, or oxo group. R₁ has a carbon number of C₈ to about C₂₄; and M is hydrogen, or metal or its equivalent (organic base such as triethanolamine, aminomethyl propanol, lysine, etc.).

Exemplary amphoteric surfactant surface treatment agents include laurylamidobetaine, stearyl amphoacetate, lauryl amphopropionate, stearyl amphopropionate, alkyl dimethylaminoacetic acid beanie, alkyl amidopropyldimethyl aminoacetic acid betaine and alkyl-N-carboxymethyl-N-hydroxyethylimidazolinium betaine. Additional examples include silane and functionalized silanes ("silanes" to "silanols" by hydrolysis): triethoxyoctylsilane, trichlorobutylsilane, trimethoxy dimethylpolysiloxyl silane. Further non-limiting examples are also described in U.S. Patent Application Publication Nos. 2006/0225616 and 2006/0286048.

Exemplary alkyl ether carboxylic acid surface treatment agents include structures and salts of [Formula II]: 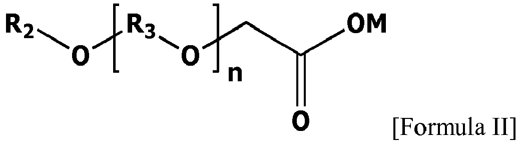
wherein R₂ is selected from the group consisting of alkyl, alkylamide, alkenyl, alkynyl, alkoxy, aryl, cycloalkyl, and arylalkyl group, all of which may be substituted by one or more hydroxy group, and may further be substituted by one or more alkoxyl, carboxyl, or oxo group. R₂ has a carbon number of C₈ to about C₂₄; R₃ includes ethylene, propylene, or butylene; n is 0 to 20; and M is hydrogen, or metal or its equivalent (organic base such as triethanolamine, aminomethyl propanol, lysine, etc.).

Exemplary acylamino acid surface treatment agents include structures and salts of [Formula III]: 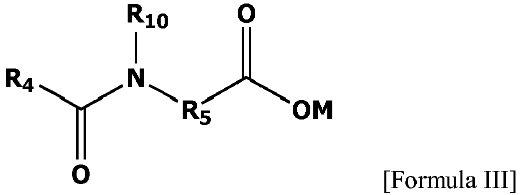
wherein R₄ and R₅ are each independently selected from the group consisting of alkyl, alkylamide, alkenyl, alkynyl, alkoxy, aryl, cycloalkyl, and arylalkyl, each of which may be substituted by one or more hydroxy group, and may further be substituted by one or more alkoxyl, carboxyl, or oxo group; R₄ has a carbon number of C₈ to about C₂₄; R₁₀ is hydrogen or methyl; and M is hydrogen, or metal or its equivalent (organic base such as triethanolamine, aminomethyl propanol, lysine, etc.).

Additional surface treatment agents include compounds having a structure represented by [Formula IV]: 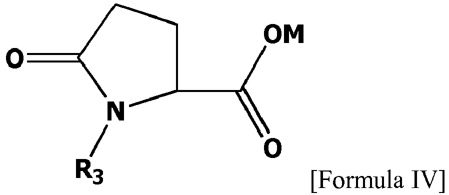
wherein R₃ is selected from the group consisting of alkyl, alkylamide, alkenyl, alkynyl, alkoxy, aryl, cycloalkyl, and arylalkyl, all of which may be substituted by one or more hydroxy group, and may further be substituted by one or more alkoxyl, carboxyl, or oxo group. R₃= C₈ to C₂₄;
M is hydrogen, or metal or its equivalent (organic base such as triethanolamine, aminomethyl propanol, lysine, etc.).

Further surface treatment agents include compounds having structure represented by [Formula V]: 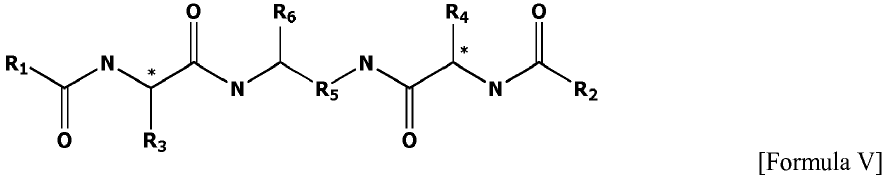
wherein R₁ and R₂ are each independently selected from an alkyl, alkylamide, alkenyl, alkynyl, alkoxy, aryl, cycloalkyl, or arylalkyl, each of which may be substituted by one or more hydroxy group, and may further be substituted by one or more alkoxyl, carboxyl, or oxo group. hydrophobic moieties, R₁, R₂ are each independently C₈ to C₂₄; R₃ and R₄ are each independently is selected from the group consisting of alkyl, alkynyl, alkenyl, and amino acid residual moieties; R₅ and R₆ are each independently is selected from the group consisting of alkyl, alkynyl, and alkenyl; at least one of R₃, R₄ and R₆ may be substituted with a carboxylic group, which is either in its acid form or a salt form (metal or its equivalent, e.g., organic base such as triethanolamine, aminomethyl propanol, lysine, etc.).

Exemplary phospholipid and alkyl ester phosphoric acid surface treatment agents include structures and salts represented by [Formula VI]: 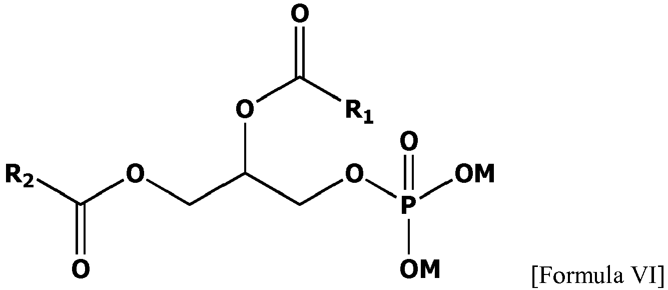
wherein R₁ and R₂ are each independently selected from the group consisting of alkyl, alkylamide, alkenyl, alkynyl, alkoxy, aryl, cycloalkyl, and arylalkyl, each of which may be substituted by one or more hydroxy group, and may further be substituted by one or more alkoxyl, carboxyl, or oxo group; R₁ and R₂ each independently have a carbon number of C₈ to about C₂₄; and M is hydrogen, or metal or its equivalent (organic base such as triethanolamine, aminomethyl propanol, lysine, etc.).

Particular embodiments of Formula VI include diester metallates and monoester dimetallates as shown below: 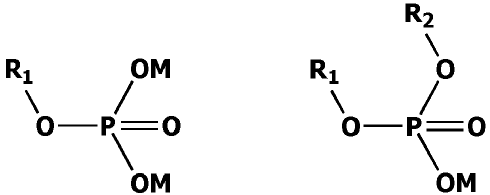
wherein R₁, R₂, and M are as defined above.

Exemplary amphoteric surface treatment agents include structures represented by [Formula VII]: 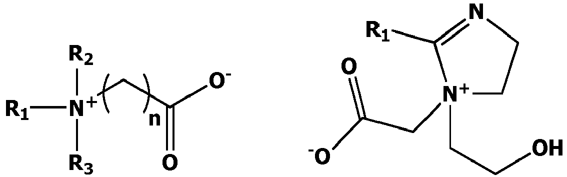
wherein R₁, R₂ and R₃ are each independently selected from the group consisting of alkyl, alkylamide, alkenyl, alkynyl, alkoxy, aryl, cycloalkyl, and arylalkyl, each of which may be substituted by one or more hydroxy group, and may further be substituted by one or more alkoxy, carboxyl, or oxo group; and R₁ has a carbon number of C₈ to about C₂₄.

Exemplary silane and silane derivative surface treatment agents include structures represented by [Formula VIII]: 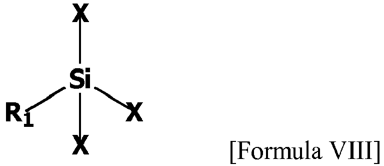
wherein R₁ is selected from the group consisting of alkyl, alkylamide, alkenyl, alkynyl, alkoxy, aryl, cycloalkyl, and arylalkyl, each of which may be substituted by one or more hydroxy group, and may further be substituted by one or more alkoxy, carboxyl, or oxo group; R₁ has a carbon number of C₈ to about C₂₄; and X is alkoxy (e.g., methoxy, ethoxy, isopropoxy, isobutoxy) or halogen (F, Cl, Br, etc.).

Additional embodiments of silane derivatives include: 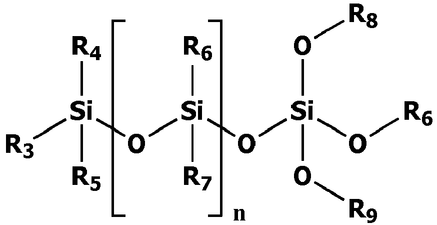
wherein R₃, R₄, R₅, R₆, R₇, R₈ and R₉ are each independently selected from the group consisting of an alkyl, alkylamide, alkenyl, alkynyl, alkoxy, aryl, cycloalkyl, and arylalkyl, each of which may be substituted by one or more hydroxy group, and may further be substituted by one or more alkoxyl, carboxyl, or oxo group; and n is 0 to 60.

Still further embodiments of surface treatment agents include compounds having a structure [Formula IX] : 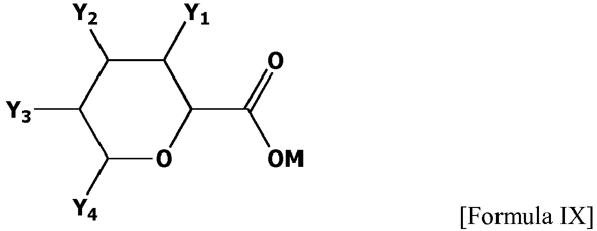
wherein Y₁, Y₂, Y₃, Y₄ are each independently selected from the group consisting of hydrogen, hydroxy, alkoxy, and oxo, wherein at least one of Y₁, Y₂, Y₃, Y₄ is a hydroxy group; and M is hydrogen, or metal or its equivalent (organic base such as triethanolamine, aminomethyl propanol, lysine, etc.).

Other embodiments of surface treatment agents include compounds having a structure [Formula X] : 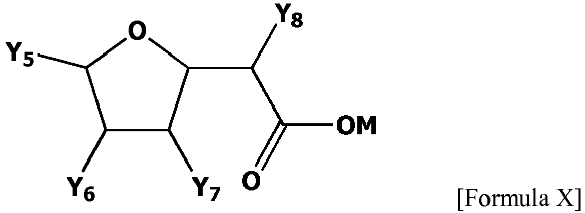
wherein Y₅, Y₆, Y₇, Y₈ are each independently selected from the group consisting of hydrogen, hydroxy, alkoxy, and oxo, wherein and at least one of Y₅, Y₆, Y₇, Y₈ is a hydroxy group;
M is hydrogen, or metal or its equivalent (organic base such as triethanolamine, aminomethyl.

Amounts of a surface treatment agent to employ in fully and partially extended color bulk powders and color bulk dispersions, intermediates thereof and production methods of the invention will vary depending upon the modification desired, the precursor product to be produced, the ultimate cosmetic, makeup, personal care or other product to be produced, or method of manufacture. For example, a surface-treatment agent may be used in an amount of at least 0.1 % by weight, based on the weight of the powder material. Surface-treatment agents are typically present in an amount ranging from about 1.0 to about 200% by weight; or, from about 1.0 to about 60% by weight; or, from about 3.0 to about 30% by weight.

The amount of a surface-treatment agent can depend, at least in part, on the specific surface area of target pigment(s), extender(s) and substrate(s). For example, for regular iron oxide pigments, 2 to about 10 parts by weight of surface-treatment agent per 100 parts of powder. For an ultrafine powder, such as silica having a large surface area, 15 to about 100 parts by weight per 100 parts of powder. Thus, the greater the surface area, the more surface-treatment agent used.

In a particular non-limiting example, for a mixture of one or more pigments with one or more substrates, a surface treatment agent is in an amount of about 0.5 to 400 parts per 100 parts of pigment and substrate. In a particular non-limiting example of a fully extended color bulk powder, the weight percent of a surface treatment agent is typically about 0.5 to 10%. In a particular non-limiting example of a fully extended color bulk dispersion, the weight percent of a surface treatment agent, based upon the total weight of the pigment(s) + substrate(s) is typically about 0.5 to 20%, or about 1.0 to 15%.

One or more pigments and one or more substrates are contacted with a surface- treatment agent, and the pigment or substrate is in turn either modified by the agent or the agent is bound to the surface of the pigment or substrate (e.g., absorbed, chemically linked or immobilized; see, for example, U.S. Patent No. 5,897,868). As an example, a substrate, pigment, or a plurality of different substrates and pigments (e.g., a mixture of different colored pigments), is contacted with a surface treatment agent which in turn becomes modified by the agent or the agent is bound to surface of the substrate and/or pigment. Surface modification of substrates and/or pigments allow the material(s) present to adhere to each other.

A surface treatment agent can be chemically immobilized or adsorbed onto the surface substrate and/or pigment. Chemical linkage or immobilization of surface-treatment agents to a substrate or pigment differs from adsorption in that surface treated material has a more uniformly chemically bound reaction product. Chemical linkage or immobilization tends to reduce movement and/or rearrangement of any material linked or attached onto the surface of the modified powder material. For example, a pigment that is linked or attached to the surface of a substrate by virtue of a surface treatment agent will have less mobility than a pigment that is attached or linked to the surface of a substrate by virtue of adsorption.

In order to facilitate or enhance immobilization of surface-treatment agents to substrate or pigment, a reaction may be created by a water soluble compound having a lipophilic or hydrophilic moiety being absorbed onto the surface of the substrate or pigment. As a non-limiting example, addition of a water-soluble salt of a polyvalent metal, such as magnesium, calcium, aluminum, titanium, zinc or a zirconium salt (e.g., zirconium sulfate or chloride), or an alkaline salt, such as a sodium, potassium, lithium, ammonium, or an amine salt, can produce a chemical linkage. The reaction provides a surface-treatment agent chemically immobilized onto the surface of the substrate or pigment particle. In contrast, coating a substrate or pigment with a surface-treatment agent involves absorbing the surface-treatment agent onto the surface of the substrate or pigment.

During treatment with a surface treatment agent, surface of one or more substrate(s) or pigment(s) become modified and in turn particles of the substrate or pigment adhere to each other. For example, small pigment particles become attached or linked to larger particles, such as substrate particles. Including a cosmetically acceptable oil (a single oil or mixture of oils) during a treatment in which substrate or pigment surface is hydrophobically modified invites oil at the same time as the particles become attached or linked to each other. Surface treatment agents and oil in combination function as a "glue" to attach or link particles, and other components optionally present, to each other. A mixture of two or more different pigments during such surface treatment results in forming color pigment composites, which are typically randomly and uniformly distributed onto the surface. Thus, oils, emulsifiers, etc., can be present in a mixture with one or more substrates and pigments when contacted with a surface treatment agent.

Following surface modification or coating, a powder material can then be admixed or blended with another (e.g., second) powder material, such as a different pigment, or substrate or extender, or another cosmetically acceptable ingredient such as an oil, emulsifier, binder, etc. The second material may or may not have been treated with a surface treatment agent. Alternatively, two or more materials (e.g., different colored pigments), can be combined or mixed together prior to contact with a surface treatment agent, such as in a aqueous slurry, and then subsequently contacted with a surface treatment agent in order to simultaneously produce two or more surface modified or coated materials. Chemical immobilization of a surface-treatment agent on materials can be facilitated by a water soluble compound having a lipophilic or hydrophilic moiety being absorbed onto the surface of the material, as set forth herein or known to the skilled artisan.

Binders can be included in color bulk powders and color bulk dispersions and employed in methods of the invention. For example, a binder, such as an oil (emollient) may but need not be present during surface treatment of a powder material such as pigment(s) or substrate(s). A binder may but need not be added prior to, during or following surface treatment of a pigment or substrate. A fully or partially extended color bulk powder or color bulk dispersion may therefore include a binder such as an oil, if desired. Although not wishing to be bound by theory, oil binders reduce movement of pigment. A binder such as an oil may but need not be added prior to or after making a fully or partially extended color bulk powder or color bulk dispersion.

"Binders" in color cosmetic industry typically refer to compounds that provide adhesive properties to material such as substrates and pigments so they remain together. Binders include oils (emollients), fats, waxes, metal soaps as a solid binder (e.g., zinc stearate, magnesium palmitate, etc.), latex emulsions, styrene, styrene butadiene, polyvinyl acetate (PVA), acrylic, acrylic-styrene, acrylic polyvinyl acetate, poylurethanes, and others acceptable for cosmetic and makeup, personal care and pharmaceutical products.

Amounts of a binders to employ in the fully and partially extended color bulk powders and color bulk dispersions, intermediates thereof and production methods of the invention will vary depending upon the precursor or intermediate product to be produced or the final or ultimate cosmetic to be produced. In a fully or partially extended color bulk powder, the weight percent of binders is typically about 0 to 25%. In a fully or partially extended color bulk dispersion, the weight percent of binders is typically about 0 to 25%. Binders may be used in an amount of at least 0 to 25% by weight. Additional oils can be added in order to increase the overall amount of binders to about 0 to 50% or 0 to 35%.

Oils include esters and waxes such as glycerides (e.g., monoglycerides, diglycerides and triglycerides), fatty acid esters, hydroxyl acid esters, dimer acid esters, other naturally derived esters (such as castor oil derivatives and vegetable-based oils, such as vegetable squalane), olive oil, camellia oil, macademia nut oil, castor oil. Wax esters include esters of higher fatty acids and higher fatty alcohols, carnauba wax, candelilla wax, jojoba oil, bees wax, lanolin. Fatty esters include isopropyl myristate, isononyl isononanoate, octyldodecyl myristate, cetyl octanoate, diisostearyl malate, caprylic- and capric triglyceride, isodecyl neopentaoate, isosteraryl neopentanoate, cholesteryl- behenyl-, octyldodecyl-lauroyl glutamate, and octylmethoxycinnamate. Petroleum and synthetic oils include hydrocarbons such as paraffins, isoparafin, petrolatum, ceresin, microcrystalline wax, squalane; silicones and derivatives thereof (such as cyclomethicone, cetyldimethicone, diphenyldimethicone, polysilicone-11, etc.), lipophilic vitamins and their derivatives (tocopherol, tocopherol acetate, tocopherol succinate, retinol, retinoic acid, retinyl parmitate, ascorbyl parmitate, etc.), lipophilic dyes, essential oils, and combinations thereof. Higher fatty acids include lauric acid, myristic acid, palmitic acid, stearic acid, isostearic acid, behenic acid, oleic acid, linoleic acid, linolenic acid, ricinoleic acid, docosa-polyenoic acid, erucic acid. Higher alcohols include behenyl alcohol, cetyl alcool, stearyl alcohol, isostearyl alcohol, octyldodecanol. Oils also include silicones: dimethyl polysiloxane ("dimethicone," available as DC 200 from Dow Coming), methylphenyl polysiloxane, cyclopentasiloxane, cetyl dimethicone, and PEG/PPG dimethicone. Other oils include mineral oil, isostearyl neopentanoate, caprylic/capric triglyceride, cetyloctanoate, diisostearyl maleate, octylmethoxycinnamate, isododecane, isononyl isononanoate, ethylhexyl methoxycinnamate, behenyl alcohol, and cholesteryl-, behenyl-, octyldodecyl-lauroyl glutamate, which produce good results. Choosing a particular oil depends in part on the product being produced.

Oil can be applied as a liquid. Oils that are not commercially available as liquids, such as ascorbyl palmitate, which is lipophilic vitamin and sold primarily as a solid, can be solubilized in liquid oil before being used as a coating oil. Suitable solubilizing oils include vitamin E acetate, caprylic/capric triglyceride, and others. Once in a liquid form, the oil may then be added to the material using conventional techniques. For example, the oil may be poured into an intake port during missing and mixed until the composition is homogeneous.

Oil, when present, is typically in an amount ranging from about 0.1 to 180% by weight, based on the weight of the substrate/pigment material. Oil, when present, can be in an amount ranging from about 1.0 to about 150% by weight; or, from about 3.0 to about 120% by weight; or, from about 5.0 to about 60% by weight; or, from about 5.0 to about 25 to 30% by weight.

The combined weight percentage of a surface-treatment agent(s) and oil, if oil is present, is typically at least about 4.0% by weight, based on the weight of the material. Typically, a combined weight percentage ranges from about 4.0 to about 1000%; or, from about 4.0 to about 500% by weight; or, from about 5.0 to about 250% by weight or, from about 8.0 to about 125% by weight or, from about 10 to about 75% by weight.

Emulsifiers, surfactants, dispersants, suspending agents, emulsion stabilizers, defoamers, thickeners and other cosmetically acceptable materials and agents can also be employed in the compositions and methods of the invention. Non-limiting examples of emulsifiers include cetyl dimethicone copolyol, polygyceryl-4 isosteatrate, glyceryl stearate, PEG-100 stearate, cetyl alcohol, dicetl phosphate, and ceteth-10 phosphate isostearic acid.

Surfactants typically include nonionic forms. Non-limiting examples of nonionic surfactants include polyoxyalkylene (PEG or/and PPG) type nonionic emulsifiers having structures: 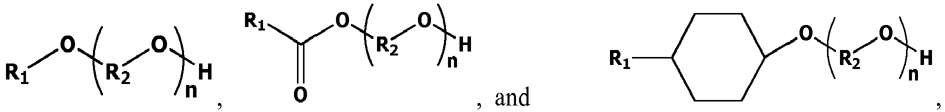
wherein R₁ is selected from the group consisting of alkyl, alkylamide, alkenyl, alkynyl, alkoxy, aryl, cycloalkyl, and arylalkyl group, each of which may be substituted by one or more hydroxy group, and may further be substituted by one or more alkoxyl, carboxyl, or oxo group. R₁ has a carbon number of C₈ to about C₂₄; R₂ is selected from the group consisting of -C₂H₄-, -C₃H₆-, and -C₄H₈-.

Further non-limiting examples of nonionic surfactants include polyhydric alcohol ester type emulsifiers wherein at least one of the hydroxy group (-OH) of a "polyol" is esterified with a fatty acid, leaving residual hydroxy groups to function as hydrophilic moieties. Residual hydroxy groups can also be modified by alkylene oxide at different polymerization number. The combination of esterified (hydrophobic) and free hydroxyl (hydrophilic) groups allows the surfactant molecule to act as an emulsifier. Non-limiting examples of polyols having different numbers of hydroxyl groups include glycerine with 3-OH's; pentaerythritol and sorbitan each with 4 -OH's; sorbitol with 6 -OH's; and sucrose with 8 -OH's. Additional non-limiting examples are shown below: 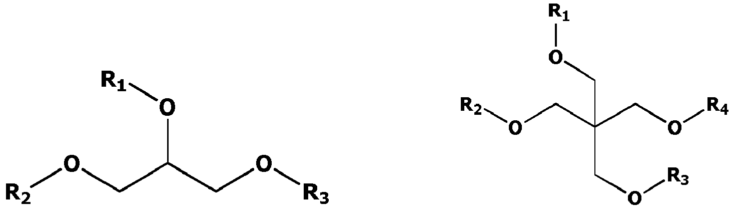 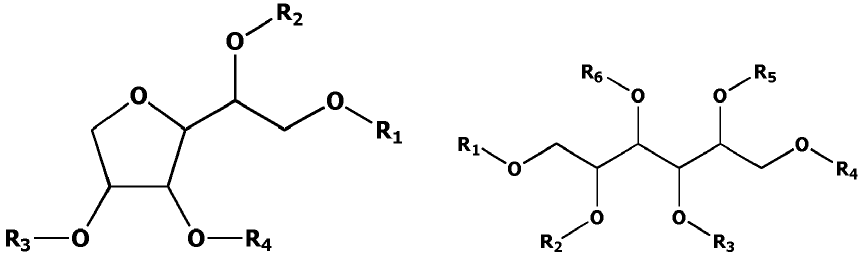 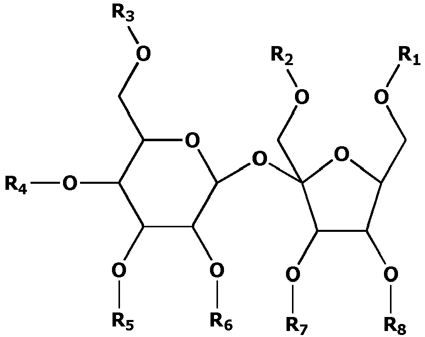
wherein R₁, R₂, R₃, R₄, R₅, R₆, R₇ and R₈ are each independently a moiety of "structure I" or "structure II" and at least one of R₁, R₂, R₃, R₄, R₅, R₆, R₇ and R₈ is "structure I".

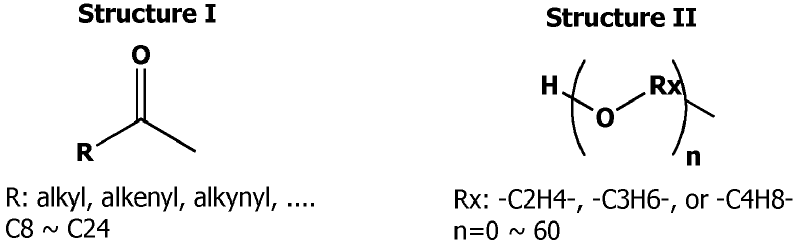

Fully and partially extended color bulk powders and fully and partially extended color bulk dispersions and intermediate products thereof can be produced as set forth herein. The invention therefore provides methods of producing (production, manufacture, preparing) fully and partially extended color bulk powders and fully and partially extended color bulk dispersions, and methods of producing (production, manufacture, preparing) intermediate products of fully and partially extended color bulk powders and fully and partially extended color bulk dispersions.

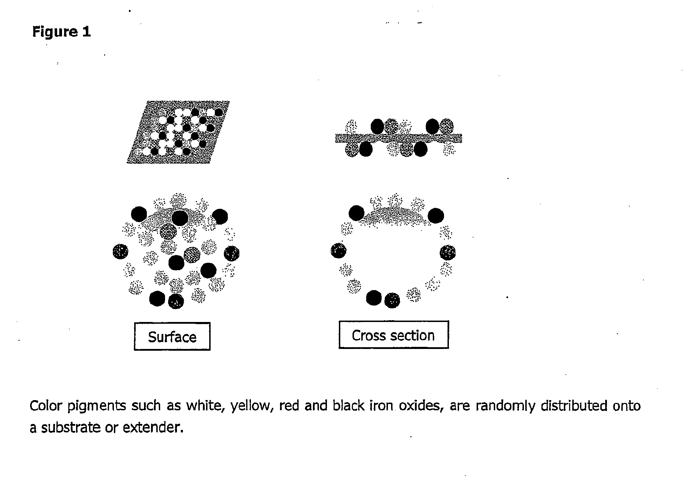

In an exemplary embodiment, a pigment (e.g., deflocculated or deagglomerated pigment) and a substrate are combined to form a mixture. The material is mixed with an aqueous solution (e.g., 50-800% water, based on pigment weight) and dispersed in a liquid slurry. The mixture may include a plurality of different pigments, the pigments in pre-determined amounts or ratios to provide a desired color, shade, hue, chroma (saturation) or lightness (see, for example, Example 2, Tables 1 and 2). A binder, such as an oil (emollient), is optionally added to the slurry (e.g., 0 to 180 parts of oil per 100 part powder). One or more surface treatment agents is then dispersed into the slurry (e.g., about 0.5 to 400 parts surface-treatment agent per 100 parts powder). The surface treatment agent(s) is chemically immobilized by a water soluble compound having a lipophilic or hydrophilic moiety being absorbed onto the surface of the substrate or pigment. As illustrated in Figure 1, the result is a "composite" substrate/extender having adhered thereto pigment particles. Different colored pigments can be adhered to substrate in an amount that reflects the predetermined amounts or ratios of pigments in the mixture. Pigments can be random and substantially uniformly distributed on the substrate, or be ordered or non-uniformly distributed on the substrate.

Following surface treatment agent immobilization, surface-modified substrate or pigment is optionally dehydrated and rinsed to remove any secondary salts and byproducts, if necessary. A filtered cake is thereby produced which may be further dehydrated to be "powder," with less than about 10% loss on drying (LOD), for example, 5% LOD, or 3% LOD.

To produce a fully extended color bulk dispersion, the filtered cake or dehydrated powder is re-dispersed into an oil phase. An oil phase may include liquid paraffin, isododecane, squalane, isononyl isononanoate, hexyl laurate, cetyl octanoate, isostearyl neopentanoate, caplylic/ capric triglyceride, tocopheryl acetate, retinyl palmitate, ascorbylpalmitate, polydimethylsiloxane, cyclopolydimethylsiloxane, ethylhexyl methoxycinnamate, PEG90 diisostearate, PEG/PPG-8/3 diisostearate, PEG/PPG-8/3 laurate, poly glyceryl-4 diisostearate, etc.

Fully and partially extended color bulk powder and fully and partially extended color bulk dispersion may be included in a cosmetic or makeup product, such as foundations (liquid foundations, loose powders, hot-pour cream foundations), lip sticks, eye shadows, eyeliners, mascaras, lotions, creams, balms, concealers, blushes, rouges, eyebrow liners, lip liners, nail polishes, and sunscreens. They may also be used in personal care (toiletry) products, such as shampoos, conditioners, lotions, deodorants, antiperspirants, moisturizers, balms, soaps and gels; or pharmaceuticals, such as ointments, salves, gels and creams. When fully and partially extended color bulk powder and fully and partially extended color bulk dispersion are in a cosmetic or makeup product or a toiletry product, other typical components used in cosmetic or toiletry products can be added, if desired. For example, cosmetic and makeup products such as lip stick and mascara will often contain various oils, waxes (e.g., Bees wax, carnauba, candellila, ozokerite, etc.) and paraffins.

Fully and partially extended color bulk powders and fully and partially extended color bulk dispersions and intermediate products thereof can be included in containers and kits, optionally including instructions for changing a shade of a fully or partially extended color bulk powder or fully or partially extended color bulk dispersion, or formulating a fully extended color bulk powder or fully extended color bulk dispersion in a cosmetic or makeup, personal care or pharmaceutical product. Specific non-limiting examples of containers and kits include a pan or bottle which contains a fully extended color bulk powder or fully extended color bulk dispersion. with at least one well therein

A container or kit optionally includes "packaging material," which refers to a physical structure housing a container or kit, or a component(s) of the container or kit. The packaging material can be made of material commonly used for such purposes. A container or kit can include a label or packaging insert with appropriate instructions, for example. Instructions may be on "printed matter," e.g., on paper or cardboard within the container or kit, or on a label affixed to the container or kit. Instructions may be provide on audio or video medium, such as an a computer readable medium, for example, floppy diskette or hard disk, optical CD such as CD- or DVD-ROM/RAM, magnetic tape, electrical storage media such as RAM and ROM and hybrids of these such as magnetic/optical storage media.

Specific non-limiting examples of containers and kits suitable for a bulk color powder and dispersion include pans, bottles, jars, vials, and tubes. Materials suitable for pans, bottles, jars, vials, and tubes include metal, glass or a polyolefin. Exemplary metals include iron (steel) and aluminum. Exemplary polyolefins include polystyrene, polypropylene, polyethylene, and polybutylene. Additional specific non-limiting examples of containers and kits include pouches, boxes, cartons and drums.

Containers and kits may be sealed. Containers and kits may include multiple (two or more) types of fully or partially extended color bulk powder, fully or partially extended color bulk dispersion or intermediate thereof of the invention. For example, a container such as a pan can include two or more wells, each of which contain a different color, shade, hue, chroma (saturation) or lightness of fully extended color bulk powder, fully extended color bulk dispersion, or an intermediate thereof. Thus, a container may contain wells, each of which have deposited thereon 1) a light pink shade fully extended color bulk powder; 2) a dark yellow shade fully extended color bulk powder; 3) a yellowish-red shade fully extended color bulk powder; and 4) a dark brown shade fully extended color bulk powder, so that when two or more of the shades are combined a different color, shade, hue, chroma (saturation) or lightness is produced. In another particular non-limiting example, a container or kit can include multiple packages each of which contains 1) a fair shade fully extended color bulk powder; 2) a golden shade fully extended color bulk powder; 3) a rose shade fully extended color bulk powder; and 4) a bark shade fully extended color bulk powder, in individual packages.

Unless otherwise defined, all technical and scientific terms used herein have the same meaning as commonly understood by one of ordinary skill in the art to which this invention relates. Although methods and materials similar or equivalent to those described herein can be used in the practice or testing of the invention, suitable methods and materials are described herein.

All publications, patents, and other references cited herein are incorporated by reference in their entirety. In case of conflict, the present specification, including definitions, will control.

As used herein, singular forms "a," "and," and "the" include plural referents unless the context clearly indicates otherwise. Thus, for example, reference to "a pigment" includes a plurality of pigments, reference to "a substrate" can include a plurality of substrates and reference to "an extender, emulsifier, surfactant, oil, etc." can include a plurality of extenders, emulsifiers, surfactants, oils, and so forth.

As used herein, all numerical values or numerical ranges include whole integers and fractions thereof within or encompassing such ranges unless the context clearly indicates otherwise. Thus, for example, reference to values such as 0 to 25% includes 0% to 5 % (i.e., 1, 2, 3, 4, 5%, or 1.1, 1.2, 1.3, 1.4, 1.5%, etc.), 10% to 20% (10, 11, 12, 13, 14%, etc., or 10.1, 10.2, 10.3, 10.4, 10.5, etc.), and so forth.

Reference to specific amount of a given ingredient or component in a color bulk powder, color bulk dispersion or intermediate thereof (e.g., a pigment, substrate, extender, oil, binder, treatment agent, etc.), such as the components and ingredients listed in Tables 1 to 3, include variations within about 1 to 20%, or 1 to 10%, or 5 to 10%, unless indicated otherwise. The term "about" typically refers to a value with about +/-1 to 10%, or 5 to 10% of the reference value.

As used herein, the term "QS," as is accustomed in the art, means a "sufficient quantity" to obtain the desired functionality. For a fragrance, the functionality is typically obtained using from about 0.05 to 1.0 wt%; for a preservative, the functionality is typically obtained using from about 0.01 to 1.0 wt%.

The invention is generally disclosed herein using affirmative language to describe the numerous embodiments. The invention also specifically includes embodiments in which particular subject matter is excluded, in full or in part. For example, one or more powder materials (e.g., substrates, pigments, pigment extender, etc.), surface treatment agents, binders (e.g., oils), emulsifiers, preservatives and fragrances can be specifically excluded in a composition or method of the invention. Thus, even though the invention is generally not expressed herein in terms of what the invention does not include, compositions and methods of the invention include embodiments in which one or more powder materials (e.g., substrates, pigments, extenders, etc.), surface treatment agents, binders (e.g., oils/emollients), emulsifiers, preservatives, fragrances, etc., are excluded.

A number of embodiments of the invention have been described. Nevertheless, it will be understood that various modifications may be made without departing from the spirit and scope of the invention. Accordingly, the following examples are intended to illustrate but not limit the scope of invention described in the claims.

### EXAMPLES

### Example 1

This example includes a description of a general procedure for preparing hydrophobically modified powder materials with fully extended color/shade, and bulk dispersion of hydrophobically modified powder materials with fully extended color/shade.

Powder material is mixed with 50 to 800% (based upon weight of the pigment) water and dispersed to make a slurry. An aqueous solution of one or more surface-treatment agents, for example, a water-soluble alkali metal salt of a fatty acid or an acyl amino acid, is added to the slurry and dispersed (0.5 to 400 parts surface-active agent per 100 parts powder). One to two chemical equivalents of a water-soluble salt of a polyvalent metal, such as an alkaline earth metal, calcium, magnesium, aluminum, titanium, zinc, or zirconium sulfate or chloride may be added to a powder material to assist in linking a functional group of the surface-treatment agent to the surface of the particles of the powder material. An oil (0 to 180 parts of oil per 100 part powder) is optionally introduced. The result is an oil coated or non oil coated, surface-modified powder material.

The oil coated or non oil coated surface-modified powder material (cake) is then optionally dehydrated, for example, using a filter press, centrifuge, freeze-drying, etc. Prior to or following dehydration, oil coated or non oil coated surface-modified powder material is optionally rinsed with water (e.g., purified, distilled or de-ionized), a water-organic solvent, and an alcohol mixture, to remove any secondary salts or byproducts, if necessary. If desired, the filtered material (cake) is further dehydrated with heat or drying to a "powder" until the moisture content reaches a desired target value, for example, 10%, or 5%, or less (e.g., 3%), loss on drying (LOD). Typical conditions for drying, which depends in part on batch size, are heating in an oven to about 100°C for about 2 hours. After cooling, the material can be further processed, for example, deflocculated. For a dispersion, filtered material (cake) or powder can be re-dispersed into an oil phase, water in oil, oil in water, or water in silicone system to produce a suspension/emulsion.

### Example 2

This example describes particular non-limiting examples of fully extended color bulk powders, and fully extended color bulk powder dispersions (water in oil emulsions).

Exemplary extended color bulk powders, and fully extended color bulk powder dispersions, a set forth in Tables 1 and 2, were prepared as follows:
Examples 1 through 5 were prepared in accordance with following procedure.
   1. Fill a tank with warm water and start high shear mixer at a speed above 6,000rpm.
   2. Add pigments and homogenize for a period of time to mix.
   3. Reduce mixing speed and add extenders, substrates and other agents while continuing to mix for a period of time to allow the components to blend.
   4. Add cosmetic oil, and mix.
   5. Add aluminum sulfate solution to initiate surface treatment of powder materials.
   6. Filter and rinse the powder with an amount of water to remove any salt byproduct (check conductivity).
   7. Dry the wet product in an oven.
   8. Pulverize the dried product with 0.020 screen, and test the powder for color extension.

Examples 14 and 17 were prepared with Example 2 through 5 powders as described above, by using a planetary mixer for 10 minutes. This powder was tested for color extension.

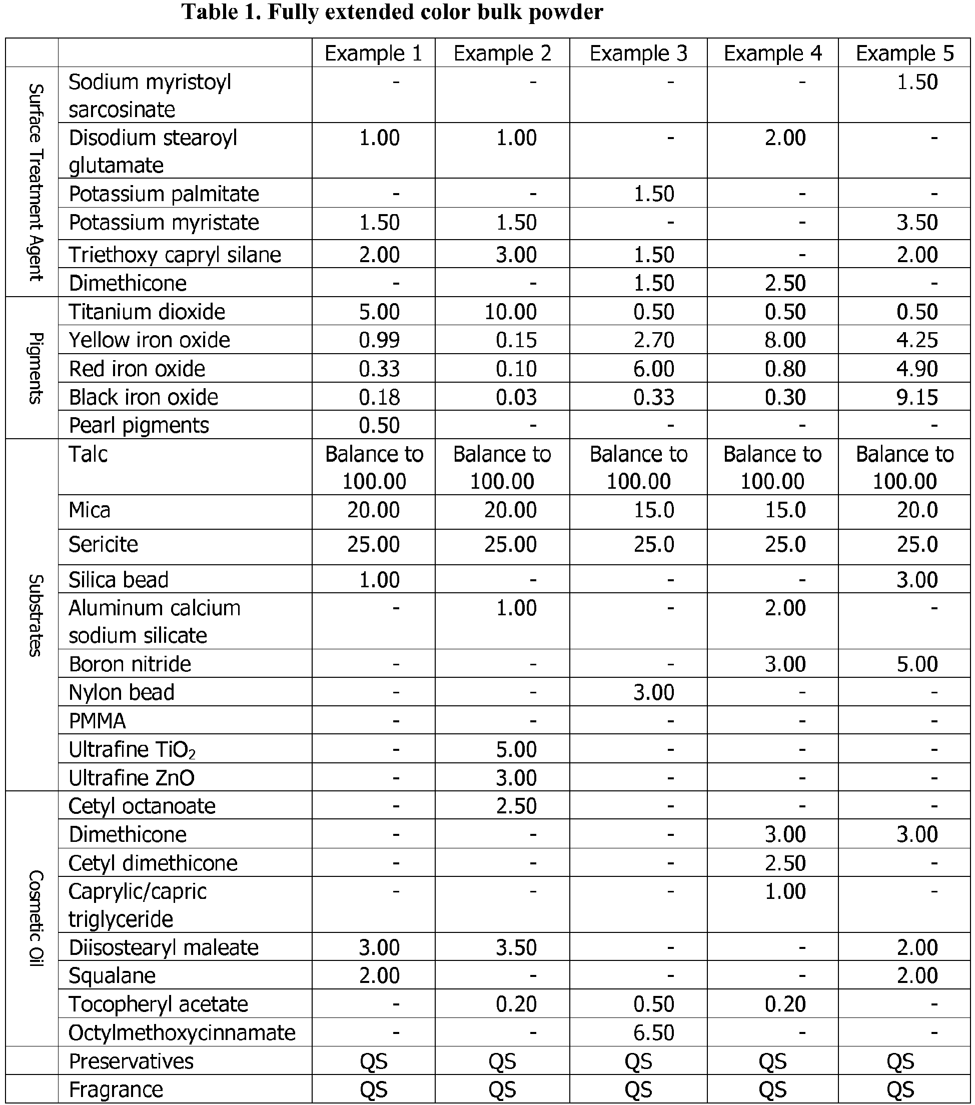

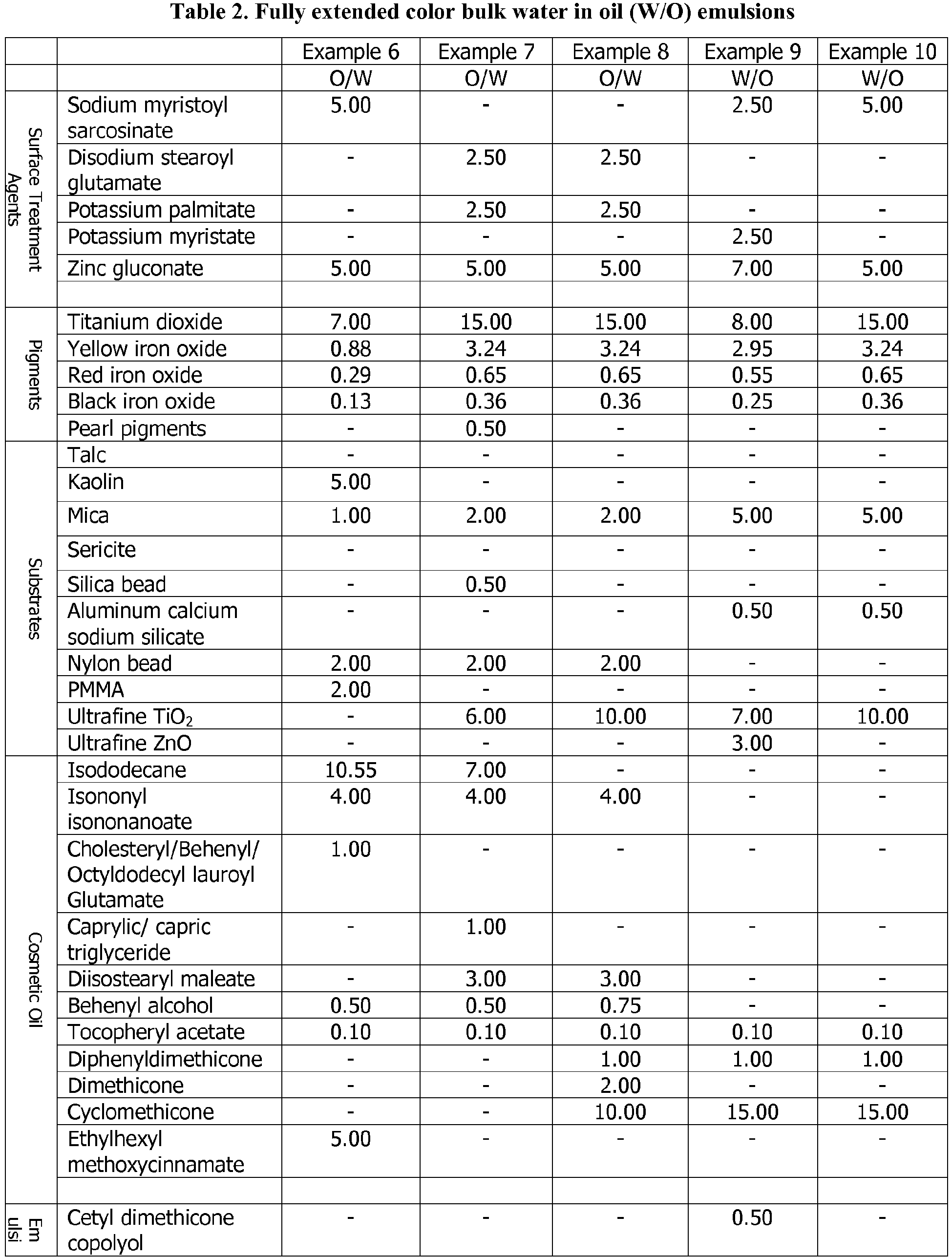

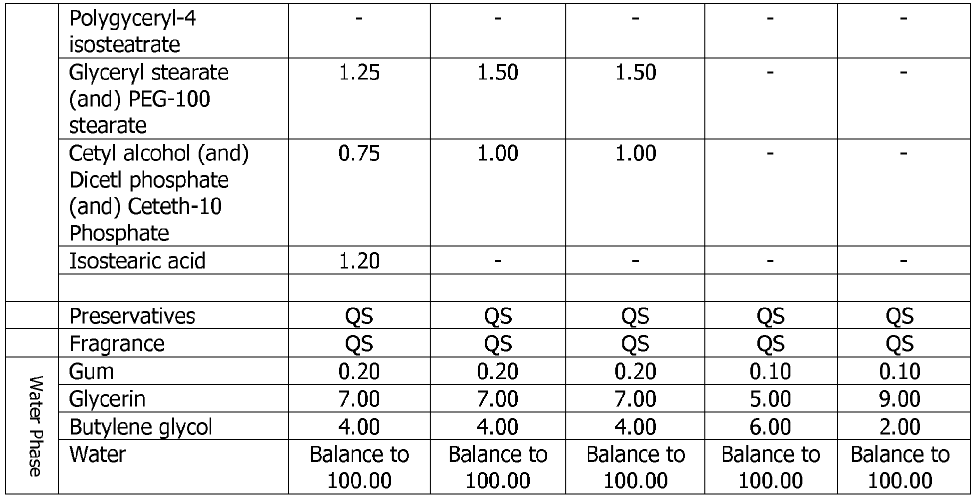

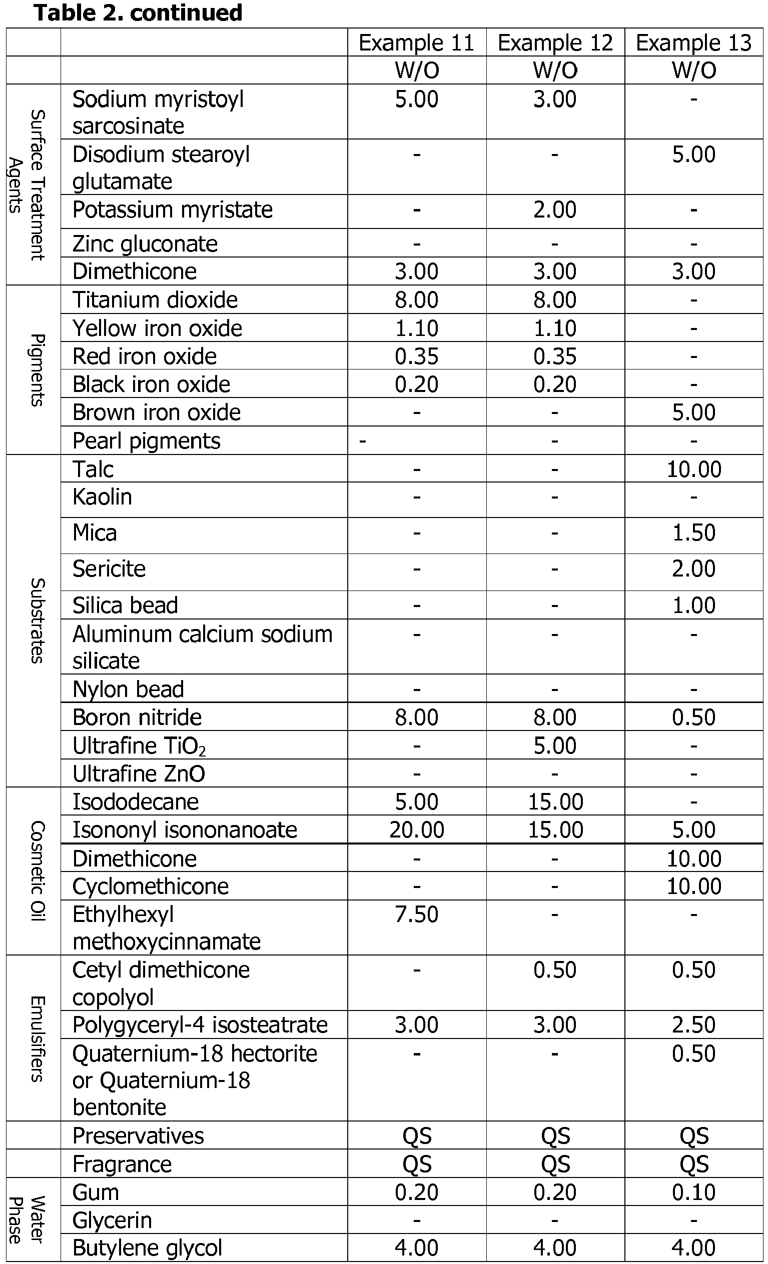

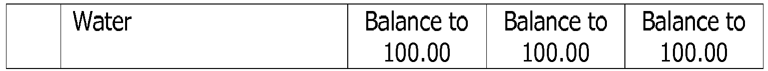

**Table 3. Examples for 4 different shades of fully extended color bulk powder, which can be blended by conventional high speed mixer to create changes in shade**

| | | **Light pink shade** | **Dark yellow shade** | **Yellowish red shade** | **Dark brown shade** |
|---|---|---|---|---|---|
| | | **Light skin tone color** | **yellow brown color** | **Red color** | **dark brown color** |
| color pigments 5to20%wt | TiO2 | **Rich 80** | Typical 28 | Typical 12 | Typical 1 |
| | yellow iron oxide | Typical 16 | **Rich 60** | Typical 24 | Typical 15 |
| | red iron oxide | Typical 3 | Typical 8 | **Rich 60** | Typical 24 |
| | black iron oxide | Typical 1 | Typical 4 | Typical 4 | **Rich 60** |
| | ultramarine, etc. | Typical QS | Typical QS | Typical QS | Typical QS |
| Substrates 0∼95% | talk, mica, sericite, kaolin, silica bead, nylon powder, functional powders | typical | typical | typical | typical |
| surface treatment 0.5∼10%wt | fatty acids, acyl amino acids, silanes, ... etc. | typical | typical | typical | typical |
| incorporated emollient 0∼25% | dimithicone, esters, ethers, glycerides, hydrocarbons, etc. | typical | typical | typical | typical |
| additional oily components 0∼25% | dimithicone, esters, ethers, glycerides, hydrocarbons, etc. | typical | typical | typical | typical |

Fully extended color bulk powders, as exemplified by four shades in Table 3, can be mixed in various amounts and proportions to produce a desired color. As an example, four shades of pigment are mixed at the ratios indicated in Table 4 below by "high speed blade mixer" or "atomizer" for certain period, which depends upon batch size. For "light skin tone," a typical mixing ratio of TiO2, yellow, red and black is about 80%, 16%, 3% and 1% (total is 100). Occasionally, a color like ultramarine is used in relatively small amounts. For "yellow brown color," yellow is used at 60% and other pigments are used at 28% for TiO2, 8% for red and 4% for black (total is 100).

After mixing, the blended fully extended color bulk powders can be pressed into pans. The color of the cake is already fully extended. Rubbing the cake surface with a sponge or finger does not substantially change the color, and the same color is applied onto the skin. Rubbing the cake surface with a moist sponge also does not substantially change the color.

**Table 4. Sample mixing ratios for fully extended color bulk powders**

| | Formula 14 | Formula 15 | Formula 16 | Formula 17 |
|---|---|---|---|---|
| Light pink shade Example 2 | 65.00 | 55.00 | 50.00 | 5.00 |
| Dark yellow shade Example 4 | 20.00 | 20.00 | 25.00 | 8.00 |
| Yellowish red shade Example 3 | 10.00 | 15.00 | 18.00 | 10.00 |
| Dark brown shade Example 5 | 5.00 | 10.00 | 7.00 | 77.00 |

**Table 5. Examples for 4 different shades of fully extended color bulk dispersion, which can be blended by regular homogenizer creating the shades**

| | | **Light pink shade** | **Dark yellow shade** | **Yellowish red shade** | **Dark brown shade** |
|---|---|---|---|---|---|
| | | **Light skin tone color** | **yellow brown color** | **Red color** | **dark brown color** |
| color pigments 5∼20%wt | TiO2 | **Rich 80** | Typical 28 | Typical 12 | Typical 1 |
| | yellow iron oxide | Typical 16 | **Rich 60** | Typical 24 | Typical 15 |
| | red iron oxide | Typical 3 | Typical 8 | **Rich 60** | Typical 24 |
| | black iron oxide | Typical 1 | Typical 4 | Typical 4 | **Rich 60** |
| | ultramarine, etc. | Typical QS | Typical QS | Typical QS | Typical QS |
| substrates 1∼95% | talk, mica, sericite, kaolin, silica bead, nilon powder, functional powders | typical | typical | typical | typical |
| surface treatment 0.5∼20%wt | fatty acids, acyl amino acids, silanes, ... etc. | typical | typical | typical | typical |
| incorporated emollient 0∼25% | dimithicone, esters, ethers, glycerides, hydrocarbons, etc. | typical | typical | typical | typical |
| additional oily components 0∼25% | dimithicone, esters, ethers, glycerides, hydrocarbons, etc. | typical | typical | typical | typical |
| Emulsifiers/ suspending agents 0∼8% | surfactants, thickeners, etc. | typical | typical | typical | typical |
| Water | | typical | typical | typical | typical |

| | | | | | |
|---|---|---|---|---|---|
| [Note] If "water"="0", it becomes "oil dispersion" If "oily components"="0", it becomes "water dispersion" If both "water" and "oily components"≠"0", it becomes a emulsion ,which can be categorized as either O/W or W/O. | | | | | |

To obtain a desired skin shade, simply mix the 4 shade dispersions in the desired proportions with an homogenizer. The color strength (saturation) can be adjusted by dilution, for example, with "oil phase" and "water phase" or addition of other cosmetic ingredients, if needed.

Fully extended color powders were compared to conventional powders, as described in U.S. Patent No. 5,968,531. Comparative Examples 1 and 2 set forth in Tables 6 and 7 were prepared as described in U.S. Patent No. 5,968,531.

**Table 6. Comparative Example 1, which corresponds to Example 3 in U.S. Patent No. 5,968,531**

| Ingredients | %wt |
|---|---|
| Example 1 in the USP_5,968,531 | 35.00 |
| SAT-TRI-77891 (Titanium Dioxides (and) Dimethicone) | 10.0 |
| SAT-Y-77492 (Iron Oxides (and) Dimethicone) | 2.50 |
| SAT-R-77491 (Iron Oxides (and) Dimethicone) | 0.80 |
| SAT-B-77499 (Iron Oxides (and) Dimethicone) | 0.30 |
| SAT-Mica (Mica (and) Dimethicone) | 7.00 |
| SAT-Sericite (Seericite (and) Dimethicone) | 20.00 |
| SAT-Talc (Talc (and) Dimethicone) | Balance to 100.00 |
| PMMA | 3.00 |
| Silica | 2.00 |
| Caprylic/ capric triglyceride | 4.00 |
| Dimethicone 20cst. | 8.20 |
| Preservative | QS |
| Fragrance | QS |

**Table 7. Comparative Example 2 consists of monochromatic color pigments of which talc is an extender**

| Ingredients | %wt |
|---|---|
| FN-TRI-77891 (Talc (and) Titanium Dioxide (and) C8-18 Fluoroalcohol Phosphate (and) Aluminum Hydroxide) | 7.15 |
| FN-Y-77492 (Talc (and) Iron Oxides (and) C8-18 Fluoroalcohol Phosphate (and) Aluminum Hydroxide) | 1.43 |
| FN-R-77491 (Talc (and) Iron Oxides (and) C8-18 Fluoroalcohol Phosphate (and) Aluminum Hydroxide) | 0.50 |
| FN-B-77499 (Talc (and) Iron Oxides (and) C8-18 Fluoroalcohol Phosphate (and) Aluminum Hydroxide) | 0.29 |
| SAT-Mica (Mica (and) Dimethicone) | 15.00 |
| SAT-Sericite (Seericite (and) Dimethicone) | 25.00 |
| SAT-Talc (Talc (and) Dimethicone) | Balance to 100.00 |
| SAT-Silica (Silica (and) Dimethicone) | 3.00 |
| Boron Nitride | 2.50 |
| Caprylic/ capric triglyceride | 2.00 |
| Diisostearyl malate | 1.00 |
| Squalane | 2.00 |
| Octylmethoxycinnamate | 6.00 |
| Tocopheryl acetate | 0.20 |
| Preservative | QS |
| Fragrance | QS |

### Color Extension Test Outline

1. Blend the loose powder using laboratory blender for certain minutes continuously (depending on the size of powder).
2. Measure the color of the blended loose powder obtaining L*, a* and b* values, which are assigned to L₀, a₀ and b₀.
3. Atomize the blended powder using a hammer mill with 0.020 inch screen. (*1* pass)
4. Measure the color of the atomized powder thereby obtaining L*, a* and b* values, which are assigned to L₁, a₁ and b₁.
5. Repeat the process 3 and 4 obtaining L*, a* and b* values, which are assigned to Lₙ, an and bₙ. (*n* pass)

### Evaluate Color Extension

6. Calculate ΔEₙ, which is [(Lₙ₊₁-Lₙ)² + (aₙ₊₁-aₙ)² + (bₙ₊₁-bₙ)²]^{1/2}, to determine the minimum pass number to obtain fully extended color.
7. If ΔE is less than about one (ΔE <1.0), the color is fully extended.

**Table 8. Color extension of Examples 1, 14 and 17, vs. Comparative Examples 1 and 2**

| Samples | ΔE₀ (no pulverizing) | ΔE₁ (1 pass) | ΔE₂ (2 pass) | ΔE₃ (3 pass) | ΔE₄ (4 pass) |
|---|---|---|---|---|---|
| Example 1 | 0.45 | - | - | - | - |
| Formula 14 (see Table 4) | 0.65 | - | - | - | - |
| Formula 17 (see Table 4) | 0.54 | - | - | - | - |
| Comparative Example 1 | 2.01 | 1.36 | 1.10 | 0.65 | - |
| Comparative Example 2 | 2.56 | 1.65 | 1.27 | 1.10 | 0.59 |

### ASPECTS OF THE DISCLOSURE:

1. A fully or partially extended color bulk powder, comprising at least one or more pigments and a substrate,
   (a) wherein the pigment is substantially deagglomerated,
   (b) wherein the pigment or substrate has a surface that has been chemically immobilized with at least one surface-treatment agent; and
   (c) wherein the pigment adheres to the substrate.
2. A fully or partially extended color bulk dispersion, comprising at least one or more pigments and a substrate,
   (a) wherein the pigment or substrate has a surface that has been chemically immobilized with at least one surface-treatment agent;
   (b) wherein the pigment adheres to the substrate; and
   (c) wherein the pigment and substrate is dispersed in a liquid medium.
3. The fully or partially extended color bulk powder or fully or partially extended color bulk dispersion of aspects 1 or 2, wherein the pigment comprises two or more different pigments, and wherein each pigment adheres to the substrate.
4. The fully or partially extended color bulk powder or fully or partially extended color bulk dispersion of aspects 1 or 2, wherein the pigment comprises three or more different pigments, and wherein each pigment adheres to the substrate.
5. The fully or partially extended color bulk powder or fully or partially extended color bulk dispersion of aspects 1 or 2, wherein the pigment comprises four or more different pigments, and wherein each pigment adheres to the substrate.
6. The fully or partially extended color bulk powder or fully or partially extended color bulk dispersion of aspects 3 to 5, wherein each pigment has a different color, shade, hue, chroma (saturation) or lightness.
7. The fully or partially extended color bulk powder or fully or partially extended color bulk dispersion of aspects 1 or 2, wherein the pigment comprises a titanium dioxide, zinc oxide, zirconium oxide, zirconium dioxide, iron oxide, ultramarine, pearl pigment, manganese violet, Prussian blue, chromium oxide, chromium hydroxides.
8. The fully or partially extended color bulk powder or fully or partially extended color bulk dispersion of aspects 1 or 2, wherein the pigment comprises rutile, anatase, ultrafine TiO₂, ultrafine ZnO, yellow iron oxide, red iron oxide, brown iron oxide, black iron oxide, ultramarine blue, ultramarine violet, ultramarine pink, mica, or titanated mica.
9. The fully or partially extended color bulk powder or fully or partially extended color bulk dispersion of aspects 1 or 2, wherein the substrate comprises clay, mica, timron super silver, a mica coated with titanium dioxide, talc, kaolin, sericite, silica, aluminum silicate, magnesium silicate, calcium sodium silicate, fumed silica, alumino-silicate, a mineral, nylon, boron nitride, an acrylate, polymethyl methacrylate (PMMA), a metal powder, ceramic powder, cotton powder, cellulose, urethane, styrene, polyolefin, polyetheylene, polyamide, zirconium, starch and starch derivatives such as aluminum starch octenylsuccinate, or calcium carbonate (chalk).
10. The fully or partially extended color bulk powder or fully or partially extended color bulk dispersion of aspects 1 or 2, wherein the surface-treatment agent comprises an acyl collagen, ether carboxylic acid, lactate, gluconate, amino acid, acyl amino acid, fatty acid, a silane, triethoxycaprylsilane, glycerol phosphate esters, methicone, dimethicone, galacturonic acid, glucarolactone, gallic acid, glucoheptanoic acid, 12-hydroxystearic acid, laurylamidobetaine, stearyl amphoacetate, lauryl amphopropionate, stearyl amphopropionate, polyethylene, sodium myristoyl sarosinate, potassium palmitate, potassium myristate, zinc gluconate, disodium stearoyl glutamate, isostearyl sebacic acid, or a combination thereof.
11. The fully or partially extended color bulk powder or fully or partially extended color bulk dispersion of aspects 1 or 2, further comprising an oil, fat, wax, fatty acid ester, fatty alcohol, or hydrocarbon.
12. The fully or partially extended color bulk powder or fully or partially extended color bulk dispersion of aspect 11, wherein the oil is a glyceride, ester, silicone or derivative thereof, a lipophilic vitamin, or a combination thereof.
13. The fully or partially extended color bulk powder or fully or partially extended color bulk dispersion of aspect 11, wherein the oil is selected from a monoglyceride, diglyceride triglyceride, a fatty acid ester, a hydroxyl acid ester, a hydrocarbon, a mineral oil, a castor oil derivative or a vegetable-based oil.
14. The fully or partially extended color bulk powder or fully or partially extended color bulk dispersion of aspect 11, wherein the oil is selected from cetyloctanoate, dimethicone, diphenyldimethicone, cyclomethicone, cetyldimethicone, polysilicone-11, caprylic- or capric-triglyceride, dimethyl polysiloxane, isostearyl neopentanoate, cetyloctanoate, diisostearyl maleate, squalane, tocopherol acetate, tocopherol (Vitamin E), retinol, retinoic acid, octylmethoxycinnamate, isododecane, isononyl isononanoate, ethylhexyl methoxycinnamate, behenyl alcohol, and cholesteryl-, behenyl-, octyldodecyl-lauroyl glutamate.
15. The fully or partially extended color bulk powder or fully or partially extended color bulk dispersion of aspect 11, wherein the oil is in an amount of about 0.1 to 25% by weight.
16. The fully or partially extended color bulk powder or fully or partially extended color bulk dispersion of aspects 1 or 2, further comprising an emulsifier.
17. The fully or partially extended color bulk powder or fully or partially extended color bulk dispersion of aspects 1 or 2, further comprising a polyglyceryl fatty acid ester, polyalkylene glycol fatty acid ester, or polyalkylene glycol alkyl ether.
18. The fully or partially extended color bulk powder or fully or partially extended color bulk dispersion of aspects 1 or 2, further comprising an emulsifier selected from Cetyl dimethicone copolyol, Polygyceryl-4 isosteatrate, Glyceryl stearate, PEG-100 stearate, Cetyl alcohol, Dicetl phosphate, Ceteth-10 Phosphate and Isostearic acid.
19. The fully or partially extended color bulk powder or fully or partially extended color bulk dispersion of aspects 1 or 2, further comprising a preservative or fragrance.
20. The fully or partially extended color bulk dispersion of aspect 2, wherein the dispersion comprises a suspension or emulsion.
21. The fully or partially extended color bulk dispersion of aspect 20, wherein the dispersion comprises a suspension or emulsion is oil, oil in water phase (o/w) water in oil phase (w/o) or water in silicone phase.
22. The fully or partially extended color bulk powder or fully or partially extended color bulk dispersion of aspects 1 or 2, wherein the color is a light skin-tone color, a dark yellow color, a yellowish-red color or a dark brown color.
23. The fully extended color bulk powder or fully extended color bulk dispersion of aspects 1 or 2, wherein the fully extended color bulk powder or fully extended color bulk dispersion exhibits little or no change in color, as indicated by a ΔE value of less than about one, as determined by the formula [(Lₙ₊₁-Lₙ)² + (aₙ₊₁-aₙ)² + (bₙ₊₁-bₙ)²]^{1/2}, after the powder or dispersion is blended.
24. The partially extended color bulk powder or partially extended color bulk dispersion of aspects 1 or 2, wherein the fully extended color bulk powder or fully extended color bulk dispersion exhibits a change in color, as indicated by a ΔE value of less than about 1.5, as determined by the formula [(Lₙ₊₁-Lₙ)² + (aₙ₊₁-aₙ)² + (bₙ₊₁-bₙ)²]^{1/2}, after the powder or dispersion is blended.
25. A fully or partially extended color bulk powder, comprising at least one pigment and at least one substrate, wherein the pigment is substantially deagglomerated, wherein the pigment or substrate has a surface that has been chemically immobilized with at least one surface-treatment agent; wherein the pigment adheres to the substrate; and wherein the fully or partially extended color bulk powder lacks one or more of a cosmetically acceptable oil, emulsifier, fat or wax.
26. The fully or partially extended color bulk powder of aspect 25, wherein the fully or partially extended color bulk powder lacks any cosmetically acceptable oil, emulsifier, fat or wax.
27. A fully or partially extended color bulk powder, comprising at least one pigment and at least one substrate, wherein the pigment is substantially deagglomerated, wherein the pigment or substrate has a surface that has been chemically immobilized with at least one surface-treatment agent; wherein the pigment adheres to the substrate; and wherein the amount of pigment is less than about 70% by weight of the total color bulk powder material.
28. The fully or partially extended color bulk powder of aspect 27, wherein the amount of pigment is less than about 60%, 50%, 40%, 30%, or 20% by weight.
29. A fully or partially extended color bulk powder, comprising at least one pigment and at least one substrate, wherein the pigment is substantially deagglomerated, wherein the pigment and substrate has a surface that has been chemically immobilized with at least one surface-treatment agent; wherein the pigment adheres to the substrate; and wherein the amount of substrate is greater than about 20% by weight of the total color bulk powder material.
30. The fully or partially extended color bulk powder of aspect 29, wherein the amount of substrate is greater than about 30%, 40%, 50%, 60%, or 70% by weight of the total color bulk powder material.
31. The fully or partially extended color bulk powder of aspects 25 to 30, wherein the pigment is substantially uniformly distributed onto the surface of the substrate.
32. The fully or partially extended color bulk powder of aspects 25 to 27, wherein the color is a light skin-tone color, a dark yellow color, a yellowish-red color or a dark brown color.
33. A fully or partially extended color bulk dispersion, comprising at least one pigment and at least one substrate, wherein the pigment or substrate has a surface that has been chemically immobilized with at least one surface-treatment agent; wherein the pigment adheres to the substrate; wherein the material is dispersed in a liquid medium; and wherein the fully or partially extended color bulk dispersion lacks one or more of a cosmetically acceptable oil, emulsifier, fat or wax.
34. The fully or partially extended color bulk dispersion of aspect 33, wherein the fully or partially extended color bulk dispersion lacks any cosmetically acceptable oil, emulsifier, fat or wax.
35. A fully or partially extended color bulk dispersion, comprising at least one pigment and at least one substrate, wherein the pigment or substrate has a surface that has been chemically immobilized with at least one surface-treatment agent; wherein the pigment adheres to the substrate; wherein the material is dispersed in a liquid medium; and wherein the amount of pigment is less than about 70% by weight of the total color bulk dispersion material.
36. The fully or partially extended color bulk dispersion of aspect 35, wherein the amount of pigment is less than about 60%, 50%, 40%, 30%, or 20% by weight of the total color bulk dispersion material.
37. A fully or partially extended color bulk dispersion, comprising at least one pigment and at least one substrate, wherein the pigment or substrate has a surface that has been chemically immobilized with at least one surface-treatment agent; wherein the pigment adheres to the substrate; wherein the material is dispersed in a liquid medium; and wherein the amount of substrate is greater than about 20% by weight of the total color bulk dispersion material.
38. The fully or partially extended color bulk powder of aspect 37, wherein the amount of substrate is greater than about 30%, 40%, 50%, 60%, or 70% by weight of the total color bulk dispersion material.
39. The fully or partially extended color bulk dispersion of aspects 33 to 38, wherein the pigment is substantially uniformly distributed onto the surface of the substrate.
40. The fully or partially extended color bulk dispersion of aspects 33 to 38, wherein the color is a light skin-tone color, a dark yellow color, a yellowish-red color or a dark brown color.
41. A cosmetic or makeup product comprising the fully or partially extended color bulk powder or fully or partially extended color bulk dispersion of aspects 1 or 2.
42. A personal care product comprising the fully or partially extended color bulk powder or fully or partially extended color bulk dispersion of aspects 1 or 2.
43. A pharmaceutical product comprising the fully or partially extended color bulk powder or fully or partially extended color bulk dispersion of aspects 1 or 2.
44. A container, said container, comprising the fully or partially extended color bulk powder or fully or partially extended color bulk dispersion of aspects 1 or 2, wherein the container has one or more of a light skin-tone color, a dark yellow color, a yellowish-red color or a dark brown color fully or partially extended color bulk powder or fully or partially extended color bulk dispersion.
45. A method for preparing fully extended color bulk powder, comprising:
   (a) providing at least one substantially deagglomerated pigment and at least one substrate;
   (b) contacting the pigment or substrate with a surface-treatment agent to produce a surface-modified pigment or substrate, thereby producing a substrate to which the pigment adheres; and
   (c) blending the material of step b) until a ΔE value of less than about one is obtained, as determined by the formula [(Lₙ₊₁-Lₙ)² + (aₙ₊₁-aₙ)² + (bₙ₊₁-bₙ)²]^{1/2}.
46. A method for preparing fully extended color bulk dispersion, comprising:
   (a) providing at least one substantially deflocculated pigment and at least one substrate;
   (b) contacting the pigment or substrate with a surface-treatment agent to produce a surface-modified the pigment or substrate, thereby producing a substrate to which the pigment adheres;
   (c) blending the material of step b) until a ΔE value of less than about one is obtained, as determined by the formula [(Lₙ₊₁-Lₙ)² + (aₙ₊₁-aₙ)² + (bₙ₊₁-bₙ)²]^{1/2}; and
   (d) dispersing the blended material in a liquid medium.
47. The method of aspects 45 or 46, further comprising: including or adding an oil, fat, wax, fatty acid ester, fatty alcohol, or hydrocarbon.
48. The method of aspect 47, wherein the oil is in an amount of about 0.1 to 25% by weight.
49. The method of aspect 47, wherein the oil is a glyceride, ester, silicone or derivative thereof, a lipophilic vitamin, or a combination thereof.
50. The method of aspect 47, wherein the oil is selected from a monoglyceride, diglyceride triglyceride, a fatty acid ester, a hydroxyl acid ester, a mineral oil, a castor oil derivative or a vegetable-based oil.
51. The method of aspect 47, wherein the oil is selected from cetyloctanoate, dimethicone, diphenyldimethicone, cyclomethicone, cetyldimethicone, polysilicone-11, caprylic- or capric-triglyceride, dimethyl polysiloxane, isostearyl neopentanoate, cetyloctanoate, diisostearyl maleate, squalane, tocopherol acetate, tocopherol (Vitamin E), retinol, retinoic acid, octylmethoxycinnamate, isododecane, isononyl isononanoate, ethylhexyl methoxycinnamate, behenyl alcohol, or cholesteryl-, behenyl-, octyldodecyl-lauroyl glutamate.
52. The method of aspect 46, wherein the liquid medium comprises water or oil.
53. The method of aspect 46, wherein the blended material is dispersed in an oil or aqueous liquid to form a suspension or an emulsion.
54. The method of aspect 53, wherein the suspension or emulsion is an oil in water phase (o/w) water in oil phase (w/o) or water in silicone phase.
55. The method of aspects 45 or 46, wherein the substrate comprises clay, mica, Timron Super Silver^{™}, a mica coated with titanium dioxide, talc, kaolin, sericite, a silica, aluminum silicate, magnesium silicate, calcium sodium silicate, fumed silica, alumino-silicate, a mineral, nylon, boron nitride, an acrylate, polymethyl methacrylate (PMMA), a metal powder, ceramic powder, cotton powder, cellulose, urethane, styrene, polyolefin, polyetheylene, polyamide, zirconium, starch and starch derivatives such as aluminum starch octenylsuccinate, or calcium carbonate (chalk).
56. The method of aspects 46 or 47, wherein the pigment comprises two or more different pigments, each of which adhere to the substrate.
57. The method of aspects 46 or 47, wherein the pigment comprises three or more different colored pigments, each of which adhere to the substrate.
58. The method of aspects 46 or 47, wherein the pigment comprises four or more different colored pigments, each of which adhere to the substrate.
59. The method of aspects 46 or 47, wherein the pigment comprises a titanium dioxide, zinc oxide, zirconium oxide, zirconium dioxide, iron oxide, ultramarine, pearl pigment, manganese violet, Prussian blue, chromium oxide, chromium hydroxides.
60. The method of aspects 46 or 47, wherein the pigment comprises rutile, anatase, ultrafine TiO₂, ultrafine ZnO, yellow iron oxide, red iron oxide, brown iron oxide, black iron oxide, ultramarine blue, ultramarine violet, ultramarine pink, mica, or titanated mica.
61. The method of aspects 46 or 47, wherein the surface-treatment agent comprises an acyl collagen, ether carboxylic acid, lactate, gluconate, amino acid, acyl amino acid, fatty acid, a silane, triethoxycaprylsilane, glycerol phosphate esters, methicone, dimethicone, galacturonic acid, glucarolactone, gallic acid, glucoheptanoic acid, 12-hydroxystearic acid, laurylamidobetaine, stearyl amphoacetate, lauryl amphopropionate, stearyl amphopropionate, polyethylene, sodium myristoyl sarosinate, potassium palmitate, potassium myristate, zinc gluconate, disodium stearoyl glutamate, isostearyl sebacic acid, or a combination thereof.
62. The method of aspects 46 or 47, further comprising adding an emulsifier.
63. The method of aspect 62, wherein the emulsifier is selected from Cetyl dimethicone copolyol, Polygyceryl-4 isosteatrate, Glyceryl stearate, PEG-100 stearate, Cetyl alcohol, Dicetl phosphate, Ceteth-10 Phosphate and Isostearic acid.
64. The method of aspects 46 or 47, further comprising adding a preservative or fragrance.
65. A method for preparing fully extended color bulk powder, comprising: providing a first color bulk powder that has a ΔE value of less than about one, as determined by the formula [(Lₙ₊₁-Lₙ)² + (aₙ₊₁-aₙ)² + (bₙ₊₁-bₙ)²]^{1/2}; and blending the first color bulk powder with a second color bulk powder that has a ΔE value of less than about one, as determined by the formula [(Lₙ₊₁-Lₙ)² + (aₙ₊₁-aₙ)² + (bₙ₊₁-bₙ)]^{1/2}, thereby preparing fully extended color bulk powder.
66. The method of aspect 65, wherein the color of the first or second color bulk powder is a light skin-tone color, a dark yellow color, a yellowish-red color or a dark brown color.
67. The method of aspect 65, wherein the first or second color bulk powder has different relative proportions of a titanium dioxide, zinc oxide, zirconium oxide, zirconium dioxide, iron oxide, ultramarine, pearl pigment, manganese violet, Prussian blue, chromium oxide, or a chromium hydroxide.
68. The method of aspect 65, wherein the first or second color bulk powder has different relative proportions of rutile, anatase, ultrafine TiO₂, ultrafine ZnO, yellow iron oxide, red iron oxide, brown iron oxide, black iron oxide, ultramarine blue, ultramarine violet, ultramarine pink, mica, or titanated mica.
69. A method for preparing fully extended color bulk dispersion, comprising: providing a first color bulk dispersion that has a ΔE value of less than about one, as determined by the formula [(Lₙ₊₁-Lₙ)² + (aₙ₊₁-aₙ)² + (bₙ₊₁-bₙ)²]^{1/2}; and blending the first color bulk dispersion with a second color bulk dispersion that has a ΔE value of less than about one, as determined by the formula [(Lₙ₊₁-Lₙ)² + (aₙ₊₁-aₙ)² + (bₙ₊₁-bₙ)²]^{1/2}, thereby preparing fully extended color bulk dispersion.
70. The method of aspect 69, wherein the color of the first or second color bulk dispersion is a light skin-tone color, a dark yellow color, a yellowish-red color or a dark brown color.
71. The method of aspect 69, wherein the first or second color bulk powder has different relative proportions of a titanium dioxide, zinc oxide, zirconium oxide, zirconium dioxide, iron oxide, ultramarine, pearl pigment, manganese violet, Prussian blue, chromium oxide, or a chromium hydroxide.
72. The method of aspect 69, wherein the first or second color bulk powder has different relative proportions of rutile, anatase, ultrafine TiO₂, ultrafine ZnO, yellow iron oxide, red iron oxid, brown iron oxide, black iron oxide, ultramarine blue, ultramarine violet, ultramarine pink, mica, or titanated mica.

## Claims

1. A fully extended color bulk powder, comprising at least one or more pigments and a substrate:
(a) wherein each of the one or more pigments is substantially deagglomerated;
(b) wherein the one or more pigments and substrate each has a surface that has been chemically immobilized with at least one surface treatment agent that has the ability to modify, alter or react with the surface of the pigment and substrate by forming chemical bonds on the surface of the pigment and substrate;
(c) wherein the one or more pigments adhere to the substrate by virtue of the at least one surface treatment agent chemically immobilized on the surface of the pigments and substrate;
(d) wherein the fully extended color bulk powder exhibits little or no change in color, as indicated by a ΔE value of less than about one, as determined by the formula [(Lₙ₊₁-Lₙ)² + (aₙ₊₁-aₙ)² + (bₙ₊₁-bₙ)²]^{1/2}, after the powder is blended only once; and
(e) wherein the amount of pigment is less than 70%, 60%, 50%, 40%, 30%, or 20% by weight of the total color bulk powder material.

2. A fully extended color bulk dispersion, comprising the fully extended color bulk powder of claim 1 dispersed in a liquid medium.

3. The fully extended color bulk powder of claim 1, wherein the amount of substrate is greater than 20%, 30%, 40%, 50%, 60%, or 70% by weight of the total color bulk powder material.

4. The fully extended color bulk powder or fully extended color bulk dispersion of claims 1-3, wherein:
(a) the one or more pigments comprises (i) two or more, (ii) three or more, or (iii) four or more different pigments; wherein each pigment adheres to the substrate; and wherein optionally each pigment has a different color, shade, hue, chroma (saturation) or lightness;
(b) the one or more pigments is selected from the group consisting of titanium dioxide, zinc oxide, zirconium oxide, zirconium dioxide, iron oxide, ultramarine, pearl pigment, manganese violet, Prussian blue, chromium oxide, chromium hydroxides, and mixtures thereof;
(c) the one or more pigments is selected from the group consisting of rutile, anatase, ultrafine TiO₂, ultrafine ZnO, yellow iron oxide, red iron oxide, brown iron oxide, black iron oxide, ultramarine blue, ultramarine violet, ultramarine pink, mica, titanated mica, and mixtures thereof;
(d) the substrate is selected from the group consisting of one or more of clay, mica, timron super silver, a mica coated with titanium dioxide, talc, kaolin, sericite, silica, aluminum silicate, magnesium silicate, calcium sodium silicate, fumed silica, alumino-silicate, a mineral, nylon, boron nitride, an acrylate, polymethyl methacrylate (PMMA), a metal powder, ceramic powder, cotton powder, cellulose, urethane, styrene, polyolefin, polyetheylene, polyamide, zirconium, starch and starch derivatives such as aluminum starch octenylsuccinate, calcium carbonate (chalk), and mixtures thereof;
(e) the at least one surface-treatment agent is selected from the group consisting of acyl collagen, ether carboxylic acid, lactate, gluconate, amino acid, acyl amino acid, fatty acid, a silane, triethoxycaprylsilane, glycerol phosphate esters, methicone, dimethicone, galacturonic acid, glucarolactone, gallic acid, glucoheptanoic acid, 12- hydroxystearic acid, laurylamidobetaine, stearyl amphoacetate, lauryl amphopropionate, stearyl amphopropionate, polyethylene, sodium myristoyl sarosinate, potassium palmitate, potassium myristate, zinc gluconate, disodium stearoyl glutamate, isostearyl sebacic acid, and combinations thereof;
(f) the fully extended color bulk powder or fully extended color bulk dispersion further comprises an oil, fat, wax, fatty acid ester, fatty alcohol, or hydrocarbon; wherein optionally:
(i) the oil is a glyceride, ester, silicone or derivative thereof, a lipophilic vitamin, or a combination thereof;
(ii) the oil is selected from a monoglyceride, diglyceride triglyceride, a fatty acid ester, a hydroxyl acid ester, a hydrocarbon, a mineral oil, a castor oil derivative or a vegetable-based oil;
(iii) the oil is selected from cetyloctanoate, dimethicone, diphenyldimethicone, cyclomethicone, cetyldimethicone, polysilicone- 11 , caprylic- or capric-triglyceride, dimethyl polysiloxane, isostearyl neopentanoate, cetyloctanoate, diisostearyl maleate, squalane, tocopherol acetate, tocopherol (Vitamin E), retinol, retinoic acid, octylmethoxycinnamate, isododecane, isononyl isononanoate, ethylhexyl methoxycinnamate, behenyl alcohol, and cholesteryl-, behenyl-, octyldodecyl-lauroyl glutamate; and/or
(iv) the oil is present in an amount of about 0.1 to 25% by weight;
(g) said fully extended color bulk powder or fully extended color bulk dispersion further comprises an emulsifier;
(h) said fully extended color bulk powder or fully extended color bulk dispersion further comprises a polyglyceryl fatty acid ester, polyalkylene glycol fatty acid ester, or polyalkylene glycol alkyl ether;
(i) said fully extended color bulk powder or fully extended color bulk dispersion further comprises an emulsifier selected from Cetyl dimethicone copolyol, Polygyceryl-4 isosteatrate, Glyceryl stearate, PEG-100 stearate, Cetyl alcohol, Dicetl phosphate, Ceteth-10 Phosphate and Isostearic acid;
(j) said fully extended color bulk powder or fully extended color bulk dispersion further comprises a preservative or fragrance;
(k) said fully extended color bulk dispersion comprises a suspension or emulsion; wherein optionally the suspension or emulsion is an oil, oil in water phase (o/w), water in oil phase (w/o) or water in silicone phase; and/or
(l) the color is a light skin-tone color, a dark yellow color, a yellowish-red color or a dark brown color.

5. A cosmetic, makeup or personal care product comprising the fully extended color bulk powder or fully extended color bulk dispersion of any one of the preceding claims.

6. A pharmaceutical product comprising the fully extended color bulk powder or fully extended color bulk dispersion of any one of claims 1 to 4.

7. A container, said container comprising the fully extended color bulk powder or fully extended color bulk dispersion of any one of claims 1 to 4.

8. A method for preparing the fully extended color bulk powder of claim 1, comprising:
(a) providing one or more substantially deagglomerated pigments and at least one substrate;
(b) contacting the one or more pigments and substrate with at least one surface-treatment agent that has the ability to modify, alter or react with the surface of the one or more pigments and substrate by forming chemical bonds on the surface of the one or more pigments and substrate to chemically immobilize the surface of the one or more pigments and substrate, thereby producing one or more pigments adhered to the substrate by virtue of the at least one surface treatment agent chemically immobilized on the surface of the one or more pigments and substrate; and
(c) blending the material of step b) until a ΔE value of less than one is obtained, as determined by the formula [(Lₙ₊₁-Lₙ)² + (aₙ₊₁-aₙ)² + (bₙ₊₁-bₙ)²]^{1/2}.

9. A method for preparing the fully extended color bulk dispersion of claim 2, comprising:
(a) providing one or more substantially deagglomerated pigments and at least one substrate;
(b) contacting the one or more pigments and substrate with at least one surface-treatment agent that has the ability to modify, alter or react with the surface of the one or more pigments and substrate by forming chemical bonds on the surface of the one or more pigments and substrate to chemically immobilize the surface of the one or more pigments and substrate, thereby producing one or more pigments adhered to the substrate by virtue of the at least one surface treatment agent chemically immobilized on the surface of the one or more pigments and substrate;
(c) blending the material of step b) until a ΔE value of less than one is obtained, as determined by the formula [(Lₙ₊₁-Lₙ)² + (aₙ₊₁-aₙ)² + (bₙ₊₁-bₙ)²]^{1/2}; and
(d) dispersing the blended material in a liquid medium.

10. The method of claims 8 or 9, wherein:
(a) said method further comprises including or adding an oil, fat, wax, fatty acid ester, fatty alcohol, or hydrocarbon; wherein optionally:
(i) the oil is in an amount of 0.1 to 25% by weight;
(ii) the oil is a glyceride, ester, silicone or derivative thereof, a lipophilic vitamin, or a combination thereof;
(iii) the oil is selected from a monoglyceride, diglyceride triglyceride, a fatty acid ester, a hydroxyl acid ester, a mineral oil, a castor oil derivative or a vegetable-based oil; and/or
(iv) the oil is selected from cetyloctanoate, dimethicone, diphenyldimethicone, cyclomethicone, cetyldimethicone, polysilicone-11, caprylic- or capric-triglyceride, dimethyl polysiloxane, isostearyl neopentanoate, cetyloctanoate, diisostearyl maleate, squalane, tocopherol acetate, tocopherol (Vitamin E), retinol, retinoic acid, octylmethoxycinnamate, isododecane, isononyl isononanoate, ethylhexyl methoxycinnamate, behenyl alcohol, or cholesteryl-, behenyl-, octyldodecyl-lauroyl glutamate;
(b) the liquid medium of claim 8 comprises water or oil;
(c) the blended material of claim 8 is dispersed in an oil or aqueous liquid to form a suspension or an emulsion; wherein optionally the suspension or emulsion is an oil in water phase (o/w), water in oil phase (w/o) or water in silicone phase;
(b) the substrate is selected from the group consisting of clay, mica, Timron Super Silver™, a mica coated with titanium dioxide, talc, kaolin, sericite, a silica, aluminum silicate, magnesium silicate, calcium sodium silicate, fumed silica, alumino-silicate, a mineral, nylon, boron nitride, an acrylate, polymethyl methacrylate (PMMA), a metal powder, ceramic powder, cotton powder, cellulose, urethane, styrene, polyolefin, polyetheylene, polyamide, zirconium, starch and starch derivatives such as aluminum starch octenylsuccinate, calcium carbonate (chalk), and mixtures thereof;
(c) the one or more pigments comprises (i) two or more, (ii) three or more, or (iii) four or more different colored pigments, each of which adhere to the substrate;
(d) the one or more pigments is selected from the group consisting of titanium dioxide, zinc oxide, zirconium oxide, zirconium dioxide, iron oxide, ultramarine, pearl pigment, manganese violet, Prussian blue, chromium oxide, chromium hydroxide, and mixtures thereof;
(e) the one or more pigments is selected from the group consisting of rutile, anatase, ultraflne TiO₂, ultrafine ZnO, yellow iron oxide, red iron oxide, brown iron oxide, black iron oxide, ultramarine blue, ultramarine violet, ultramarine pink, mica, titanated mica, or mixtures thereof;
(f) the at least one surface-treatment agent is selected from the group consisting of acyl collagen, ether carboxylic acid, lactate, gluconate, amino acid, acyl amino acid, fatty acid, a silane, triethoxycaprylsilane, glycerol phosphate esters, methicone, dimethicone, galacturonic acid, glucarolactone, gallic acid, glucoheptanoic acid, 12- hydroxystearic acid, laurylamidobetaine, stearyl amphoacetate, lauryl amphopropionate, stearyl amphopropionate, polyethylene, sodium myristoyl sarosinate, potassium palmitate, potassium myristate, zinc gluconate, disodium stearoyl glutamate, isostearyl sebacic acid, and combinations thereof;
(g) the method further comprises adding an emulsifier; wherein optionally the emulsifier is selected from Cetyl dimethicone copolyol, Polygyceryl-4 isosteatrate, Glyceryl stearate, PEG-100 stearate, Cetyl alcohol, Dicetl phosphate, Ceteth-10 Phosphate and Isostearic acid; and/or
(j) the method further comprises adding a preservative or fragrance.
